(19) Europäisches Patentamt
European Patent Office
Office européen des brevets

(11) EP 2 889 299 A1

# (12) EUROPEAN PATENT APPLICATION

(43) Date of publication:
**01.07.2015 Bulletin 2015/27**

(21) Application number: **13199575.5**

(22) Date of filing: **24.12.2013**

(51) Int Cl.:
***C07D 491/00*** *(2006.01)* ***C09B 56/00*** *(2006.01)*
***C09B 62/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Paris Sciences et Lettres - Quartier Latin**
**75005 Paris (FR)**

(72) Inventors:
- **MALLET, Jean-Maurice**
  **94400 Vitry sur Seine (FR)**
- **COLLOT, Mayeul**
  **67400 Illkirch Graffenstaden (FR)**

(74) Representative: **Icosa**
**83 avenue Denfert-Rochereau**
**75014 Paris (FR)**

## (54) Fluorescent red emitting functionalizable pH probes

(57) The present invention relates to pH probes of formula I:

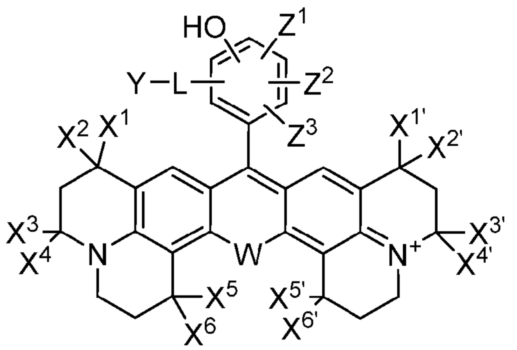

wherein:
**W** represents O, NH, N(alkyl), Si(alkyl)$_2$, CH$_2$, CH(alkyl), C(alkyl)$_2$; preferably
**W** represents O;
**X¹** and **X¹'** represent both H, both Me or both -CH$_2$-SO$_3$H;
**X²** and **X²'** represent both H or both Me;
**X³** and **X³'** represent both H or both Me;
**X⁴** and **X⁴'** represent both H or both Me;
**X⁵** and **X⁵'** represent both H or both Me;
**X⁶** and **X⁶'** represent both H or both Me;
**Z¹**, **Z²** and **Z³**, represent each independently H, electron withdrawing group or electron donating group,
**L** represents a single bound or a linker,
**Y** represents a reactive group, or a bioactive group,
and a process for manufacturing said compounds. The invention further relates to the use of the compounds of the invention as pH probes.

EP 2 889 299 A1

## Description

### FIELD OF INVENTION

The present invention relates to pH probes and use thereof for pH measurement, especially fluorescent red-emitting functionalizable pH probes. The invention further relates to a process for manufacturing said pH probes and to method of pH determination comprising the use of the pH probes of the invention.

### BACKGROUND OF INVENTION

pH probes are mainly used in biological research and medical diagnostics. They may be used in studies including cytotoxicity tests, flow cytometry, dose response analysis and cellular imaging using confocal microscopy. Analyzed samples may be for example live cells or biological fluids comprising endogenous host cell proteins, buffers solutions and environmental samples. Especially, pH probes may be used to monitor pH changes and events directly or indirectly associated with a change of pH. For example, monitoring of pH may give indications relative to the health of the cells, ion transportation methods or internalization pathways. One of the most common uses of pH probes in cell biology is the imagery of acidic domains such as lysosomes which exhibit a pH range of 4.5 to 5.5.

pH probes comprise a pH sensing moiety and a detection moiety. For fluorescent pH probes, the detection moiety is a fluorophore as represented on figure 1. The interaction between the sensing moiety and the fluorophore depends on the state of the sensor (protonated/deprotonated), and results in a measurable change in fluorescence (Han and Burgess, Chem. Rev., 2010, 110, 2709-2728).

Fluorescent pH probes may be based on three different on/off mechanisms for the generation of the fluorescent response to pH variations. A first mechanism is based on a spirolactame ring opening reaction that renders the fluorophore fluorescent. pH probes comprising a spirolactame and a rhodamine as fluorophore and thus emitting red fluorescence were disclosed by Tian et al., Dyes and Pigments, 2012, 95, 112-115; Zhang et al., Analyst, 2009, 134, 367-371). The initial spirolactam is non-fluorescent, however, upon protonation, the system is ring opened yielding a highly fluorescent species. The drawback of probes based on this first mechanism is that the pKa is hardly tunable since the structure of these probes is very specific.

A second mechanism implies fluorescence control by the ionization of phenolic hydroxyl groups in a fluorophore, such as for example in fluorescein, carboxyfluorescein, Oregon Green®, SNARF® and SNAFL®. The most commonly used emit green fluorescence, such as fluoresceine, carboxyfluorescein or Oregon Green®. Others emit red fluorescence such as SNARF® and SNAFL®, involving a rhodamine fluorophore. These red pH probes present the drawback to have low quantum yields. Further pH probes based on BODIPY as fluorophore were also proposed, most of them emitting green fluorescence (Baruah et al., J. Org. Chem., 2005, 70, 4152-4157; Qin et al., ChemPhysChem, 2005, 6, 2343-2351). To the knowledge of the Applicant, a single red-emitting pH probe implying ionization of phenolic hydroxyl groups in a BODIPY fluorophore was disclosed, based on styryl BODIPY (Qin et al., J. Photochem. Photobiol. A: Chem., 2006, 183, 190-197). The degree of ionization of this type of pH probes increases upon lowering the acidity of the environment, it becomes more fluorescent upon pH increase. However, for biological applications, it is preferred that fluorescence decrease upon pH increase, in order to image acidic domains. Therefore, pH probes based on this second mechanism are not well suitable for cell imaging.

A third mechanism relies on the "PET effect" (Photoinduced Electron Transfert). In this system, when the sensor is analyte-free, an electron is transferred to the orbital vacated by excitation upon absorption of a photon. Such an electron transfer prevents the excited molecule from emission transition, leading to the quenching of the fluorescence of the probe. Conversely, when the sensor is bound (i.e. upon protonation), the nature and energy of the pair's orbital is changed and stops the PET. As a result, the fluorophore responds to a pH change by a modification in the relative fluorescence intensity and the fluorescence decay time. Because protonation cancels the quenching, the PET-based sensors become more fluorescent as pH decreases.

Typical pH probes based on this third mechanism include an amino group (aliphatic or aromatic) as a sensing moiety along with a fluorophore. Examples of PET-based pH probes include LysoSensor probes, which contain dimethylamino group as detection moiety and CypHer® 5E dye having an indolenine detection moiety. Patent application WO2008/076524 discloses PET-based pH probes comprising an aniline sensing moiety and a rhodamine as fluorophore. These systems based on amino sensors present the drawback to be sensible to oxidation, which may lead to probe destruction by photooxidation. Moreover, amines protonate in very acidic conditions and it may thus be difficult to tune the pH amino-probe to reach pKa of biological interest.

PET effect is thus very interesting to generate fluorescent response to pH variations. However, known fluorescent pH probes involving this mechanism are not optimal and there is a need for new PET-based fluorescent pH probes.

Most pH probes combine sensing moiety with fluorescein derivatives and hence emit yellow/green fluorescence. However, the increasing use of cells transfected with fluorescent proteins (FPs), especially eGFP (enhanced Green

Fluorescent Protein), of FP-expressing transgenic mice for targeting identified subpopulations of cells, together with the advent of optical techniques for purposes other than imaging, require the development of new pH probes emitting at longer-wavelength.

The demand for longer-wavelength and higher signal-to-noise chemical pH probes is accentuated by the recent trend toward all-optical manipulation and recording. Photopharmacology, photochemical uncaging, and optogenetics all use near-ultraviolet or short visible wavelengths that further restrain the part of the visible spectrum available for pH imaging.

Therefore, to be valuable for biological pH imaging, new pH probes should be bright and operate in spectral windows outside the yellow/green.

Another important parameter when discussing pH probes is their affinity for protons, described in terms of acid dissociation constant ($K_a$), using the logarithmic form pKa ($-\log_{10}K_a$), to indicate the acidity of a compound. A high acidity means a lower proton affinity and hence a lower pKa value.

Providing a set of pH probes with a wide range of pKa values is interesting in order to vary fields of pH measurement and to cater for all possible cellular environments.

There is thus a need for sets of pH probes with wide pKa values to enable biologists to target specific pH-dependent processes.

Moreover, it is essential to provide functionalizable pH probes in order to widen their fields of use. For example, conjugation of pH probes to bioactive molecules would enable to target the probe for biological applications. Anchoring pH probes to solid support may also be interesting to develop for example suitable detection kits. The introduction of a functionalizable arm of the pH probe should not be deleterious to fluorescence properties and should be taken into account for pKa modulation.

Taken all together, there is thus a need for new fluorescent pH probes based on PET effect, which have good optical properties and operate in spectral windows outside the yellow/green, having tunable pKa values and being functionalizable. By "good optical properties" it is especially understood a good molar extinction coefficient, a good quantum yield and a good dynamic range and being functionalizable. The pH probe should enable detecting pH changes in biological systems.

The present invention thus relates to new red emitting pH probes comprising a X-rhodamine moiety as fluorophore and a phenolic group as sensing moiety. Especially, the invention relates to a compound of formula I:

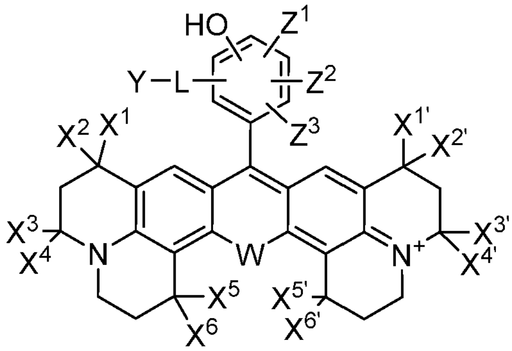

wherein **W, L, Y, $Z^1$, $Z^2$, $Z^3$, $X^1$, $X^2$, $X^3$, $X^4$, $X^5$, $X^6$, $X^{1'}$, $X^{2'}$, $X^{3'}$, $X^{4'}$, $X^{5'}$** and **$X^{6'}$** are as defined below.

To the knowledge of the Applicant, PET-based functionalizable pH probes comprising a phenolic moiety as pH sensor and X-rhodamine as detecting moiety were never disclosed in the art.

Phenol has a native pKa value of 9.95. This value decreases with the introduction of the X-rhodamine group, which is an electron-withdrawing group. Introducing electron donating and/or withdrawing groups on the phenolic sensor of the red-emitting pH probes of the invention enables tuning their pKa.

The X-Rhodamine chromophore is similarly bright as fluorescein, but is red-shifted in both excitation (574 nm) and emission (600 nm) and present the advantage to be insensible to pH in the biological pH range and to be photostable.

The process of manufacturing of the pH probe of the invention presents the advantage to require a limited number of steps. The sensor need only contain an aldehyde group to act as the building block for X-rhodamine formation via simple two-step process. The method of synthesis is flexible enough to allow the introduction of pKa modulating substituents.

The versatility of the indicators of the invention is further increased since they may be functionalized with numerous molecular tools such as for example an antibody, a benzylguanine (SNAP tag) or a peptide to facilitate specific sub-cellular targeting. Especially, the presence of the functionalizable arm enables to introduce moieties suitable to control the localization of the probe, to make it enter into the cell and to avoid its accumulation in mitochondria once

entered in to the cell.

Moreover, the pH probes of the invention may be combined with activity indicators emitting in the green-yellow spectral band, to allow multiplexed imaging and quantitative pH monitoring.

The pH probes of the invention further present the advantage to be specific to their intended function and not affected by other biologically important species such as free metal ions, such as for example $Zn^{2+}$, $Mg^{2+}$, $Ca^{2+}$, $Fe^{3+}$ and $Mn^{2+}$.

Therefore, the red emitting pH probes of the invention are powerful and versatile probes with tunable pKa.

## DEFINITIONS

In the present invention, the following terms have the following meanings:

- **"about"** preceding a figure, means plus or less 10% of the value of said figure.

- **"acylamino"** refers to the groups -NRC(O)alkyl, -NRC(O)cycloalkyl, -NRC(O)cycloalkenyl, -NRC(O)alkenyl, -NRC(O)alkynyl, -NRC(O)aryl, -NRC(O)heteroaryl and -NRC(O)heterocyclic, wherein R is hydrogen or alkyl.

- **"alkoxy"** refers to any group -O-alkyl, wherein alkyl is as defined above. Suitable alkoxy groups include for example methoxy, ethoxy, n-propoxy, isopropoxy, n-butoxy, f-butoxy, sec-butoxy, and n-pentoxy.

- **"alkyl"** refers to a hydrocarbyl radical of formula $C_nH_{2n+1}$ wherein n is a number greater than or equal to 1. Generally, alkyl groups of this invention comprise from 1 to 12 carbon atoms, preferably from 1 to 6 carbon atoms. Alkyl groups may be linear or branched and may be substituted as indicated herein. Suitable alkyl groups include methyl, ethyl, propyl (n-propyl, i-propyl, n- butyl), butyl (i-butyl, s-butyl and t-butyl), pentyl and its isomers (e.g. n-pentyl, iso-pentyl), and hexyl and its isomers (e.g. n-hexyl, iso-hexyl).

- **"alkylthio"** refers to any group -S-alkyl wherein alkyl is as define above.

- **"alkyne"** refers to a class of monovalent unsaturated hydrocarbyl groups, wherein the unsaturation arises from the presence of one or more carbon-carbon triple bonds. Alkynyl groups typically, and preferably, have the same number of carbon atoms as described above in relation to alkyl groups. Non limiting examples of alkynyl groups are ethynyl, 2- propynyl, 2-butynyl, 3-butynyl, 2-pentynyl and its isomers, 2-hexynyl and its isomers-and the like.

- **"AM ester"** or **"AM"** as used therein, by itself or as part of another group, refers to an acetoxymethyl ester of a carboxylic acid.

- **"amino"** refers to the group $-NH_2$. **"alkylamino"** refers to the group -NHR wherein R is alkyl. **"dialkylamino"** refers to the group -NRR' wherein R and R' are alkyl.

- **"aminocarbonyl"** refers to the group -C(O)NR'R" wherein R' and R" are independently selected from the group comprising hydrogen, alkyl, alkenyl, alkynyl, aryl, cycloalkyl, cycloalkenyl, heteroaryl and heterocyclic, and wherein R' and R" are optionally joined together with the nitrogen bound thereto to form a heterocyclic or substituted heterocyclic group, such as for example a substituted piperazine.

- **"aminothiocarbonyl"** refers to the group -C(S)NR'R" wherein R' and R" are independently selected from the group comprising hydrogen, alkyl, alkenyl, alkynyl, aryl, cycloalkyl, cycloalkenyl, heteroaryl and heterocyclic, and wherein R' and R" are optionally joined together with the nitrogen bound thereto to form a heterocyclic or substituted heterocyclic group, such as for example a substituted piperazine.

- **"aryl"** refers to a polyunsaturated, aromatic hydrocarbyl group having a single ring (i.e. phenyl) or multiple aromatic rings fused together (e.g. naphtyl) or linked covalently, typically containing 5 to 20 atoms; preferably 6 to 12, wherein at least one ring is aromatic. The aromatic ring may optionally include one to two additional rings (either cycloalkyl, heterocyclyl or heteroaryl) fused thereto. Aryl is also intended to include the partially hydrogenated derivatives of the carbocyclic systems enumerated herein. Non- limiting examples of aryl comprise phenyl group, the biphenyl group, the 1-naphthyl group, the 2-naphthyl group, the tetrahydronaphthyl group, the indanyl group and the binaphthyl group.

- **"dextran"** refers to a complex, branched glucan (i.e. a polysaccharide made of many glucose molecules) composed

of chains of varying lengths. The straight chain consist of α-1,6 glycosidic linkages between glucose molecules, while braches begin from α-1,3 linkages.

- **"electron donating group"** refers to a substituent with lone pairs that is adjacent to an aromatic ring and increases electron density on the ring through a resonance donating effect. Examples of electron donating groups are: $-O^-$, $-NR_2$, $-NH_2$, -OH, -OR, -SH, -SR, -NH(C=O)R, -O(C=O)R, -R, -Ph, $-CH=CR_2$ wherein R preferably stands for alkyl, though it may be substituted in any reasonable way which does not transform the electronic effect of alkyl from donating to withdrawing.

- **"electron withdrawing group"** refers to groups where the atom adjacent to the aromatic pi system has a formal positive charge, or has a δ positive charge (for example, due to being connected to more electronegative atoms). Examples of electron withdrawing groups are: -halo, -(C=O)H, -(C=O)R, -COOR, -COOH, $-CF_3$, -CN, $-SO_3H$, $-NH_3^+$, $-NR_3^+$, $-NO_2$ wherein R preferably stands for alkyl, though it may be substituted in any reasonable way which does not transform the electronic effect of alkyl from withdrawing to donating.

- **"fatty acid"** refers to a carboxylic acid with a long aliphatic chain, which is either saturated or unsaturated. Preferably the aliphatic chain has a number of carbon atoms ranging from 4 to 28. In a preferred embodiment, fatty acids are derived from triglycerides or phospholipids.

- **"fluorophore"** refers to a molecule or a portion of molecule which exhibits fluorescence.

- **"halo"** refers to fluoro, chloro, bromo, or iodo. Preferred halo groups are fluoro and chloro.

- **"linker"** refers to a single covalent bond or a moiety comprising series of stable covalent bonds, the moiety often incorporating 1-40 plural valent atoms selected from the group consisting of C, N, O, S and P, that covalently attach a reactive group or bioactive group to the phenolic sensor group of the pH probe of the invention. The number of plural valent atoms in a linker may be, for example, 0, 1, 2, 3, 4, 5, 6, 7, 8, 9, 10, 20, 25, 30 or a larger number up to 40 or more. A linker may be linear or non-linear; some linkers have pendant side chains or pendant functional groups (or both). Examples of such pendant moieties are hydrophilicity modifiers, for example solubilising groups like, e.g. sulfo ($-SO_3H$ or $-SO_3^-$).

In one embodiment, L is composed of any combination of single, double, triple or aromatic carbon-carbon bonds, carbon-nitrogen bonds, nitrogen-nitrogen bonds, carbon-oxygen bonds and carbon-sulfur bonds. Linkers may by way of example consist of a combination of moieties selected from alkyl, -C(O)NH-, -C(O)O-, -NH-,- S-, -O-, -C(O) -, $-S(O)_n-$ where n is 0, 1 or 2; -O-, 5- or 6- membered monocyclic rings and optional pendant functional groups, for example sulfo, hydroxy and carboxy.

The reactive group may be reacted with a substance reactive therewith, whereby the linker becomes bonded to a bioactive group. In this case, the linker typically contains a residue of a reactive group (such as for example the carbonyl group of an ester or a triazolo group resulting from a click reaction between an azide and an alkyne).

- **"probe"** refers to a compound that emits light to produce an observable detectable signal. A **"fluorescent pH probe"** refers to a compound whose fluorescent spectrum or intensity is affected by pH.

- **"reactive group"** refers to a group capable of reacting with another chemical group to form a covalent bond, i.e. is covalently reactive under suitable reaction conditions, and generally represents a point of attachment for another substance. The reactive group is a moiety on the compounds of the present invention that is capable of chemically reacting with a functional group on a different compound to form a covalent linkage. Reactive groups generally include nucleophiles, electrophiles and photoactivatable groups.

- **"rhodamine"** refers to a family of related chemical compounds which are heterotricyclic and fluorescent and based on fluorone. Rhodamine refers for example to Rhodamine 6G, Rhodamine B or X-rhodamine. According to a preferred embodiment, the red-emitting fluorophore of the pH probe of the invention is a rhodamine, more preferably X-rhodamine.

- **"salt"** of the compounds of the invention includes the acid addition and base salts thereof. Suitable acid addition salts are formed from acids which form non-toxic salts. Non-limiting examples include the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts. Suitable base salts are formed from bases which form non-toxic salts. Non-limiting examples include the aluminium, arginine, benzathine, calcium, choline, diethylamine, diolamine, glycine, lysine, magnesium, meglumine, olamine, potassium, sodium, tromethamine, 2-(diethylamino)ethanol, ethanolamine, morpholine, 4- (2- hydroxyethyl)morpholine and zinc salts. Hemisalts of acids and bases may also be formed, for example, hemisulphate and hemicalcium salts. Preferred, pharmaceutically acceptable salts include hydrochloride/chloride, hydrobromide/bromide, bisulphate/sulphate, nitrate, citrate, and acetate.

- **"sample"** refers to any material that may contain an analyte of interest or cells. According to one embodiment, the sample is a live cell or a biological fluid that comprises endogenous host cells. Alternatively, the sample may be a buffer solution or an environmental sample for which pH determination is needed. The sample may be in an aqueous solution, a viable cell culture or immobilized on a solid or semi-solid surface such as a polyacrylamide gel, membrane blot or on a microarray.

Unless indicated otherwise, the nomenclature of substituents that are not explicitly defined herein are arrived at by naming the terminal portion of the functionality followed by the adjacent functionality toward the point of attachment. For example, the substituent "arylalkyl" refers to the group (aryl)-(alkyl)-.

## DETAILED DESCRIPTION

### Compounds

The present invention relates to a pH probe comprising a red-emitting fluorophore and a pH sensing moiety, as schematically represented on figure 1.

In a preferred embodiment, the present invention relates to a pH probe comprising a X-rhodamine moiety as fluorophore and a phenolic moiety as pH sensor. Especially, the invention relates to a compound of formula I:

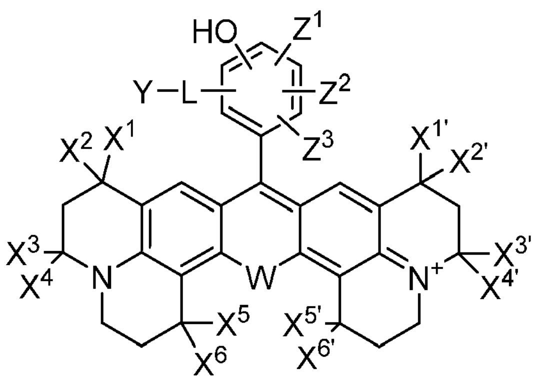

and salts thereof, wherein:

**W** represents O, NH, N(alkyl), $Si(alkyl)_2$, $CH_2$, CH(alkyl), $C(alkyl)_2$; preferably
**W** represents O;
**$X^1$** and **$X^{1'}$** represent both H, both Me or both $-CH_2-SO_3H$;
**$X^2$** and **$X^{2'}$** represent both H or both Me;
**$X^3$** and **$X^{3'}$** represent both H or both Me;
**$X^4$** and **$X^{4'}$** represent both H or both Me;
**$X^5$** and **$X^{5'}$** represent both H or both Me;
**$X^6$** and **$X^{6'}$** represent both H or both Me;
**$Z^1$, $Z^2$** and **$Z^3$**, represent each independently H, electron withdrawing group or electron donating group, preferably **$Z^1$, $Z^2$** and **$Z^3$**, represent each independently H, alkyl, aryl, amino, alkylamino, dialkylamino, thiol, alkylthio, hydroxy, alkoxy, acylamino, (carboxyl ester)oxy, halo, $NO_2$, CN, $-SO_3H$, -COOH, ester, aldehyde, ketone;
**L** represents a single bound or a linker, preferably the linker is selected from aminocarbonyl, alkylaminocarbonyl, aminothiocarbonyl, alkyloxycarbonyl, alkyl, aryl, alkylaryl, arylalkyl, alkoxy, polyethylene glycol (PEG), polypropylene glycol (PPG), peptide; or a combination thereof; optionally interrupted or terminated by -O-, -S-, $-SO_2$ or a combination

thereof; optionally additionally comprising a residue of a reactive group through which L is bounded to **Y;**
**Y** represents:

- a reactive group selected from the group comprising $N_3$, amino, alkylamino, COOH, amide, maleimide, alkyne, SH, OH, ester, activated ester, activated carboxylic acid, halo, nitro, nitrile, isonitriles, acrylamide, aldehyde, ketone, acetals, ketals, anhydride, glutaric anhydride, succinic anhydride, maleic anhydride, thiocyanate, isothiocyanate, isocyanate, hydrazide, hydrazines, hydrazones, ethers, oxides, cyanates, diazo, diazonium, sulfides, disulfides, sulfoxides, sulfones, sulfonic acids, sulfinic acids, sulfates, sulfenic acids, amidines, imides, imidates, nitrones, hydroxylamines, oximes, hydroxamic acids, thiohydroxamic acids, allenes, ortho esters, sulfites, enamines, ynamines, ureas, pseudoureas, semicarbazides, carbodiimides, carbamates, imines;

- a bioactive group selected from amino acid, peptide, protein, antibody, enzyme, polysaccharide, dextran, benzylguanine, lipid, lipid assembly, fatty acid, nucleoside, nucleotide, oligonucleotide, hapten, aptamer, ligand, substrate, biotin, avidin, synthetic polymer, polymeric microparticle, nanoparticle, fluorophore, chromophore, radioisotope, macrocyclic complexes of radioisotope, and combinations thereof.

According to an embodiment, activated ester refers for example to N-hydroxysuccinimide ester, N-hydroxyglutarimide ester or maleimide ester.

According to an embodiment, activated carboxylic acid refers for example to acid anhydride or acid halide.

According to one embodiment, $X^1$, $X^2$, $X^3$, $X^4$, $X^5$, $X^6$, $X^{1'}$, $X^{2'}$, $X^{3'}$, $X^{4'}$, $X^{5'}$ and $X^{6'}$ represent H; or $X^1$, $X^2$, $X^{1'}$, $X^{2'}$, $X^5$, $X^6$, $X^{5'}$ and $X^{6'}$ represent Me and $X^3$, $X^4$, $X^{3'}$ and $X^{4'}$ represent H; or $X^1$, $X^h$, $X^3$, $X^4$, $X^{3'}$ and $X^{4'}$ represent Me and $X^2$, $X^{2'}$, $X^5$, $X^6$, $X^{5'}$ and $X^{6'}$ represent H; or $X^1$ and $X^{1'}$ represent $-CH_2-SO_3H$, $X^3$, $X^4$, $X^{3'}$ and $X^{4'}$ represent Me and $X^2$, $X^{2'}$, $X^5$, $X^6$, $X^{5'}$ and $X^{6'}$ represent H.

According to one embodiment, $X^1$, $X^2$, $X^3$, $X^4$, $X^5$, $X^6$, $X^{1'}$, $X^{2'}$, $X^{3'}$, $X^{4'}$, $X^{5'}$ and $X^{6'}$ represent H.

According to one embodiment, $X^1$, $X^2$, $X^{1'}$, $X^{2'}$, $X^5$, $X^6$, $X^{5'}$ and $X^{6'}$ represent Me and $X^3$, $X^4$, $X^{3'}$ and $X^{4'}$ represent H.

According to one embodiment, $X^1$, $X^{1'}$, $X^3$, $X^4$, $X^{3'}$ and $X^{4'}$ represent Me and $X^2$, $X^{2'}$, $X^5$, $X^6$, $X^{5'}$ and $X^{6'}$ represent H.

According to one embodiment, $X^1$ and $X^{1'}$ represent $-CH_2-SO_3H$, $X^3$, $X^4$, $X^{3'}$ and $X^{4'}$ represent Me and $X^2$, $X^{2'}$, $X^5$, $X^6$, $X^{5'}$ and $X^{6'}$ represent H.

According to one embodiment, $Z^1$, $Z^2$ and $Z^3$, represent each independently H, halo, alkyl or alkoxy, preferably H, Cl, Br, methyl or OMe.

Advantageously, the choice of $Z^1$, $Z^2$ and $Z^3$ enable to tune the pKa of the compound of formula I.

According to one embodiment, **Y** represents a reactive group selected from the group comprising $N_3$, amino, alkylamino, COOH, amide, maleimide, alkyne, SH, OH, ester, activated ester, activated carboxylic acid, halo, nitro, nitrile, isonitriles, acrylamide, aldehyde, ketone, acetals, ketals, anhydride, glutaric anhydride, succinic anhydride, maleic anhydride, thiocyanate, isothiocyanate, isocyanate, hydrazide, hydrazines, hydrazones, ethers, oxides, cyanates, diazo, diazonium, sulfides, disulfides, sulfoxides, sulfones, sulfonic acids, sulfinic acids, sulfates, sulfenic acids, amidines, imides, imidates, nitrones, hydroxylamines, oximes, hydroxamic acids thiohydroxamic acids, allenes, ortho esters, sulfites, enamines, ynamines, ureas, pseudoureas, semicarbazides, carbodiimides, carbamates, imines.

According to a preferred embodiment, **Y** represents a reactive group selected from the group comprising $N_3$, alkyne, amino, alkylamino, COOH, amide, maleimide, SH, OH, ester, activated ester, activated carboxylic acid, preferably $N_3$ or alkyne.

According to one embodiment, **Y** represents a bioactive group selected from amino acid, peptide, protein, antibody, enzyme, polysaccharide, dextran, benzylguanine, lipid, lipid assembly, fatty acid, nucleoside, nucleotide, oligonucleotide, hapten, aptamer, ligand, substrate, biotin, avidin, synthetic polymer, polymeric microparticle, nanoparticle, fluorophore, chromophore, radioisotope, macrocyclic complexes of radioisotope, and combinations thereof.

According to a preferred embodiment, **Y** represents a bioactive group selected from amino acid, peptide, protein, antibody, enzyme, polysaccharide, dextran, benzylguanine, lipid, lipid assembly, fatty acid, ligand, substrate, biotin, avidin, fluorophore, chromophore.

According to one embodiment, in compound of formula **I,**
**L** represents a linker, preferably a linker selected from aminocarbonyl, alkylaminocarbonyl, aminothiocarbonyl, alkyloxycarbonyl, alkyl, aryl, alkylaryl, arylalkyl, alkoxy, polyethylene glycol (PEG), polypropylene glycol (PPG), peptide or a combination thereof; optionally interrupted or terminated by -O-, -S-, $-SO_2$ or a combination thereof; and
**Y** represents a reactive group selected from the group comprising $N_3$, amino, alkylamino, COOH, amide, maleimide, alkyne, SH, OH, ester, activated ester, activated carboxylic acid, halo, nitro, nitrile, isonitriles, acrylamide, aldehyde, ketone, acetals, ketals, anhydride, glutaric anhydride, succinic anhydride, maleic anhydride, thiocyanate, isothiocyanate, isocyanate, hydrazide, hydrazines, hydrazones, ethers, oxides, cyanates, diazo, diazonium, sulfides, disulfides, sulfoxides, sulfones, sulfonic acids, sulfinic acids, sulfates, sulfenic acids, amidines, imides, imidates, nitrones, hydroxylamines, oximes, hydroxamic acids, thiohydroxamic acids, allenes, ortho esters, sulfites, enamines, ynamines, ureas, pseudoureas, semicarbazides, carbodiimides, carbamates, imines.

According to a preferred embodiment, in compound of formula I,
**L** represents an aminocarbonyl group, preferably an alkylaminocarbony, a piperazineaminocarbonyl or an oxycarbonylpiperazineaminocarbonyl group; and **Y** represents $N_3$ or alkyne.

According to one embodiment, in compound of formula I,
**L** represents a linker, preferably a linker selected from aminocarbonyl, alkylaminocarbonyl, aminothiocarbonyl, alkyloxycarbonyl, alkyl, aryl, alkylaryl, arylalkyl, alkoxy, polyethylene glycol (PEG), polypropylene glycol (PPG), peptide or a combination thereof; optionally interrupted or terminated by -O-, -S-,- $SO_2$ or a combination thereof; optionally additionally comprising a residue of a reactive group through which L is bounded to **Y;** and
**Y** represents a bioactive group selected from amino acid, peptide, protein, antibody, enzyme, polysaccharide, dextran, benzylguanine, lipid, lipid assembly, fatty acid, nucleoside, nucleotide, oligonucleotide, hapten, aptamer, ligand, substrate, biotin, avidin, synthetic polymer, polymeric microparticle, nanoparticle, fluorophore, chromophore, radioisotope, macrocyclic complexes of radioisotope, and combinations thereof.

According to a preferred embodiment, in compound of formula I,
**L** represents an aminocarbonyl group, preferably an alkylaminocarbony, a piperazineaminocarbonyl or an oxycarbonylpiperazineaminocarbonyl group; optionally additionally comprising a residue of a reactive group through which L is bounded to **Y;** such as for example a triazolo group; wherein **L** represents; and
**Y** represents a bioactive group selected from amino acid, peptide, protein, antibody, enzyme, polysaccharide, dextran, benzylguanine, lipid, lipid assembly, fatty acid, ligand, substrate, biotin, avidin, fluorophore, chromophore.

The compound of the invention presents the advantage of being functionalizable or functionalized. According to one embodiment, functionalization is performed through Y when Y represents a reactive group.

According to one embodiment, the pH probe of the invention is functionalized by a lipophilic group such as an AM (acetoxymethoxy) or acetate ester, which allows for entry across the live cell membrane.

According to an embodiment, the compound of the invention is of formula Ia:

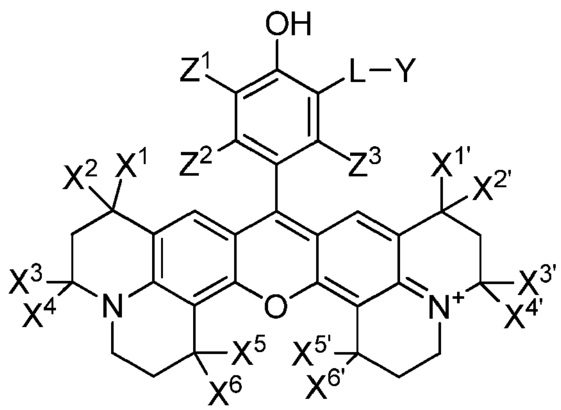

and salts thereof, wherein **L, Y, $Z^1$, $Z^2$**, **$Z^3$**, **$X^1$**, **$X^2$, $X^3$**, **$X^4$**, **$X^5$**, **$X^6$**, **$X^{1'}$**, **$X^{2'}$**, **$X^{3'}$**, **$X^{4'}$**, **$X^{5'}$** and **$X^{6'}$** are as defined above.

According to a specific embodiment, in formula Ia, **$Z^1$, $Z^2$** and **$Z^3$** represent hydrogen atoms.

According to an embodiment, the compound of the invention is of formula Ia-1:

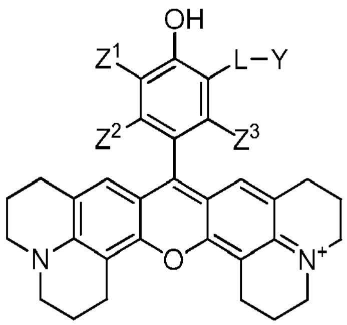

and salts thereof, wherein **$Z^1$, $Z^2$**, **$Z^3$**, **L** and **Y** are as defined above.

According to an embodiment, the compound of the invention is of formula Ib:

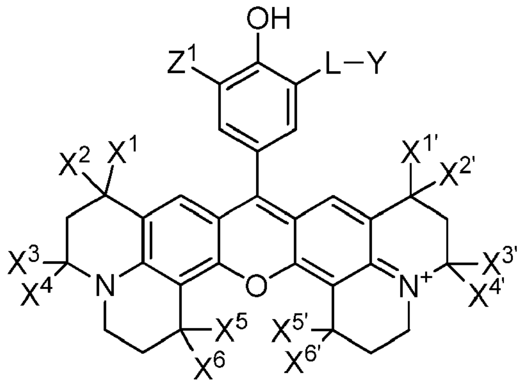

and salts thereof, wherein **L, Y, $Z^1$, $X^1$**, **$X^2$**, **$X^3$**, **$X^4$, $X^5$**, **$X^6$**, **$X^{1'}$**, **$X^{2'}$**, **$X^{3'}$, $X^{4'}$**, **$X^{5'}$** and **$X^{6'}$** are as defined above.

According to a preferred embodiment, the compound of the invention is of formula Ib-1, corresponding to compound of formula Ib wherein **$X^1$**, **$X^2$, $X^3$**, **$X^4$, $X^5$, $X^6$**, **$X^{1'}$**, **$X^{2'}$**, **$X^{3'}$, $X^{4'}$**, **$X^{5'}$** and **$X^{6'}$** are hydrogen atoms.

According to a specific embodiment, in formulae Ib or Ib-1, $Z^1$ is an hydrogen atom. According to a specific embodiment, in formulae Ib or Ib-1, $Z^1$ is $NO_2$, CN, $-SO_3H$ or $-SO_3^-$, halo; preferably halo is F, Cl, Br.

According to an embodiment, the compound of the invention is of formula Ic:

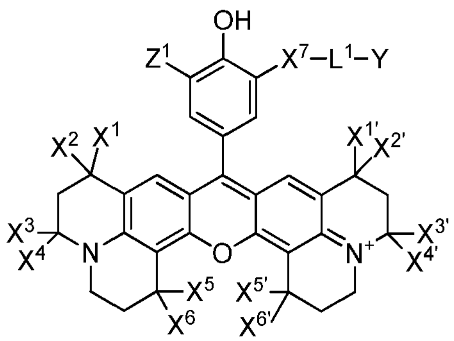

and salts thereof, wherein:

- **$Z^1$, $X^1$**, **$X^2$**, **$X^3$**, **$X^4$**, **$X^5$**, **$X^6$**, **$X^{1'}$**, **$X^{2'}$**, **$X^{3'}$, $X^{4'}$**, **$X^{5'}$**, **$X^{6'}$** and Y are as defined above;
- **$X^7$** represents a single bound or $-SO_2-$, -O-, -S-, -(C=O)N-, -(C=O)-; and
- **$L^1$** represents a single bound, alkyl, aryl, alkylaryl, arylalkyl, or a combination thereof; optionally additionally comprising a residue of a reactive group through which L is bounded to **Y.**

According to an embodiment, the compound of the invention is of formula Ic-1, corresponding to compound of formula Ic wherein **$X^1$**, **$X^2$, $X^3$, $X^4$**, **$X^5$, $X^6$**, **$X^{1'}$, $X^{2'}$, $X^{3'}$, $X^{4'}$**, **$X^{5'}$** an, **$X^{6'}$** are hydrogen atoms.

According to a specific embodiment, in formulae Ic or Ic-1, $Z^1$ is an hydrogen atom. According to another specific embodiment, in formulae Ic or Ic-1, $Z^1$ is $NO_2$, CN, $-SO_3H$ or $-SO_3^-$, halo; preferably halo is F, Cl, Br.

According to an embodiment, the compound of the invention is of formula Id:

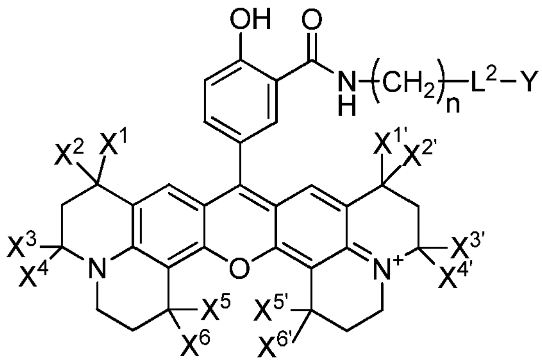

and salts thereof, wherein:

$X^1$, $X^2$, $X^3$, $X^4$, $X^5$, $X^6$, $X^{1'}$, $X^{2'}$, $X^{3'}$, $X^{4'}$, $X^{5'}$, $X^{6'}$ and **Y** are as defined above;
$L^2$ represents a single bound or a residue of a reactive group through which Y is bounded to the rest of the compound; and
**n** represents an integer ranging from 1 to 8, preferably from 1 to 6, more preferably 1, 2, 3, or 4.

According to an embodiment, the compound of the invention is of formula Id-1, corresponding to compound of formula Id wherein $X^1$, $X^2$, $X^3$, $X^4$, $X^5$, $X^6$, $X^{1'}$, $X^{2'}$, $X^{3'}$, $X^{4'}$, $X^{5'}$ an, $X^{6'}$ are hydrogen atoms.
In a preferred embodiment, in formulae Id or Id-1, Y represents $N_3$ or alkyne, preferably $N_3$ or ethynyl.
In a specific embodiment, in formulae Id or Id-1, $L^2$ represents a triazolo group.
According to an embodiment, the compound of the invention is of formula Ie:

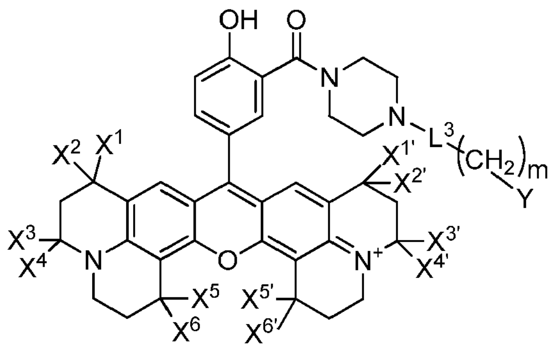

and salts thereof, wherein:

$X^1$, $X^2$, $X^3$, $X^4$, $X^5$, $X^6$, $X^{1'}$, $X^{2'}$, $X^{3'}$, $X^{4'}$, $X^{5'}$, $X^{6'}$ and **Y** are as defined above;
$L^3$ represents a single bound or an alkyloxycabonyl; and
**m** represents an integer ranging from 1 to 8, preferably from 1 to 6, more preferably 1, 2, 3, or 4.

According to an embodiment, the compound of the invention is of formula Ie-1, corresponding to compound of formula Ie wherein $X^1$, $X^2$, $X^3$, $X^4$, $X^5$, $X^6$, $X^{1'}$, $X^{2'}$, $X^{3'}$, $X^{4'}$, $X^{5'}$ an, $X^{6'}$ are hydrogen atoms.
In a preferred embodiment, in formulae Ie or Ie-1, Y represents $N_3$ or alkyne, preferably $N_3$ or ethynyl.
According to one embodiment, the compound of the invention is selected from the group comprising compounds of the table below:

| Compound n° | Formula |
|---|---|
| **I-1** | 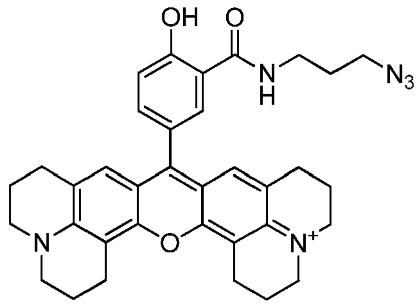 |
| **I-2** | 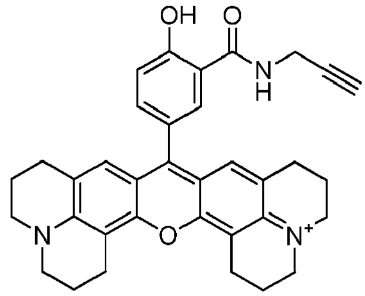 |
| **I-3** | 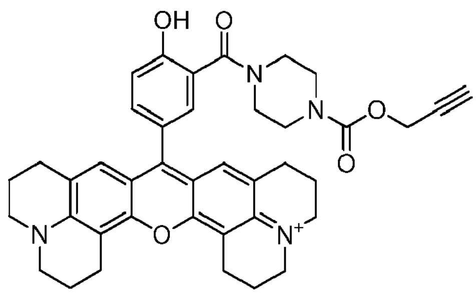 |
| **I-4** | 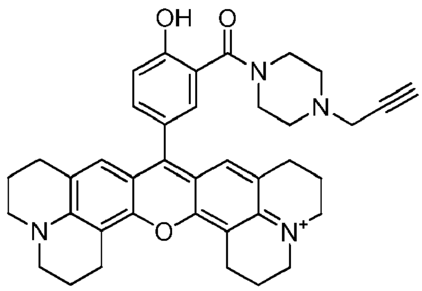 |
| **I-5** | 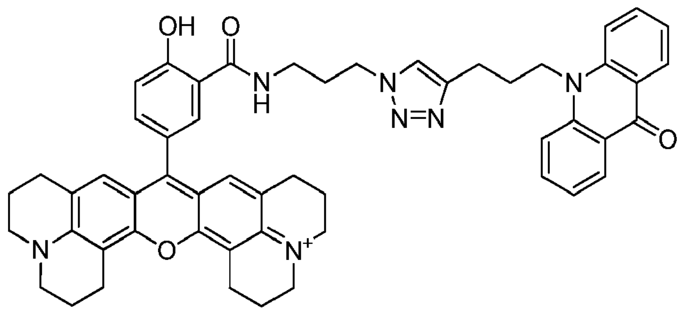 |

(continued)

| Compound n° | Formula |
|---|---|
| **I-6** | 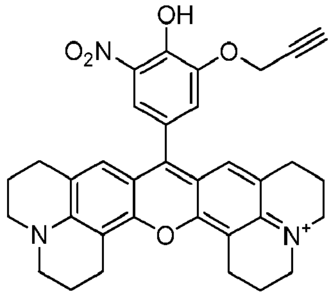 |

### *pH properties*

According to one embodiment, the compound of the invention has a pKa ranging from 2 to 12, preferably from 4 to 10, more preferably from 4 to 9.6.

Depending on substituents on the phenolic sensor, it is advantageously possible to modulate the pKa of the pH probe. In a first embodiment, the compound of the invention has a pKa ranging from 3 to 5. In a second embodiment, the compound of the invention has a pKa ranging from 5 to 8. In a third embodiment, the compound of the invention has a pKa ranging from 8 to 12.

### *Fluorescence*

According to an embodiment, the compound of the invention is fluorescent. Preferably the compound of the invention is a red fluorescent probe. According to preferred embodiment, the compound of the invention emits at a wavelength of more than 600 nm.

In an embodiment, the compound of the invention has a quantum yield ranging from 25 to 80%. Quantum yield may be measured by as described in "Measurement of Fluorescence Quantum Yields", Michael W. Allen, Thermo Fisher Scientific, Madison, WI, USA.

In an embodiment, the compound of the invention may be characterized by its dynamic range. Dynamic range reflects the importance of fluorescence increase between ON and OFF forms of the probe. Dynamic range may be measured by spectrofluorimetry with solutions of increasing pH. In an embodiment, the compound of the invention has a dynamic range ranging from 30 to 100, preferably from 80 to 98.

## <u>Process for manufacturing</u>

The compounds of the invention may be prepared using any suitable reactions known by those skilled in the art.

The invention further relates to a process for manufacturing the compounds of the invention. Especially, the invention relates to a process for manufacturing a compound of formula I:

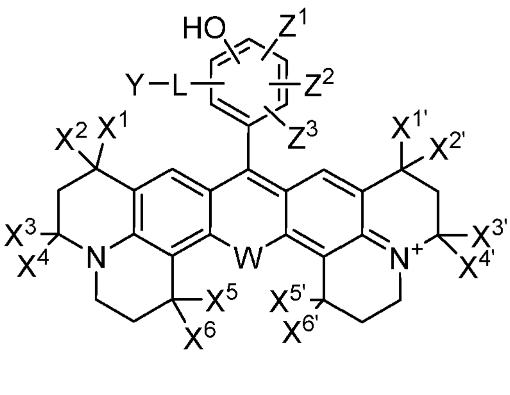

and salts thereof, wherein:

**W** represents O, NH, N(alkyl), Si(alkyl)$_2$, $CH_2$, CH(alkyl), C(alkyl)$_2$; preferably
**W** represents O;
**X¹** and **X¹'** represent both H, both Me or both $-CH_2-SO_3H$;
**X²** and **X²'** represent both H or both Me;
**X³** and **X³'** represent both H or both Me;
**X⁴** and **X⁴'** represent both H or both Me;
**X⁵** and **X⁵'** represent both H or both Me;
**X⁶** and **X⁶'** represent both H or both Me;
**Z¹, Z²** and **Z³**, represent each independently H, electron withdrawing group or electron donating group, preferably **Z¹, Z²** and **Z³**, represent each independently H, alkyl, aryl, amino, alkylamino, dialkylamino, thiol, alkylthio, hydroxy, alkoxy, acylamino, (carboxyl ester)oxy, halo, $NO_2$, CN, $-SO_3H$, -COOH, ester, aldehyde, ketone;
**L** represents a single bound or a linker, preferably the linker is selected from aminocarbonyl, alkylaminocarbonyl, aminothiocarbonyl, alkyloxycarbonyl, alkyl, aryl, alkylaryl, arylalkyl, alkoxy, polyethylene glycol (PEG), polypropylene glycol (PPG), peptide or a combination thereof; optionally interrupted or terminated by -O-, -S-, $-SO_2$ or a combination thereof; optionally additionally comprising a residue of a reactive group through which L is bounded to **Y;**
**Y** represents:

- a reactive group selected from the group comprising $N_3$, amino, alkylamino, COOH, amide, maleimide, alkyne, SH, OH, ester, activated ester, activated carboxylic acid, halo, nitro, nitrile, isonitriles, acrylamide, aldehyde, ketone, acetals, ketals, anhydride, glutaric anhydride, succinic anhydride, maleic anhydride, thiocyanate, isothiocyanate, isocyanate, hydrazide, hydrazines, hydrazones, ethers, oxides, cyanates, diazo, diazonium, sulfides, disulfides, sulfoxides, sulfones, sulfonic acids, sulfinic acids, sulfates, sulfenic acids, amidines, imides, imidates, nitrones, hydroxylamines, oximes, hydroxamic acids, thiohydroxamic acids, allenes, ortho esters, sulfites, enamines, ynamines, ureas, pseudoureas, semicarbazides, carbodiimides, carbamates, imines;
- a bioactive group selected from amino acid, peptide, protein, antibody, enzyme, polysaccharide, dextran, benzylguanine, lipid, lipid assembly, fatty acid, nucleoside, nucleotide, oligonucleotide, hapten, aptamer, ligand, substrate, biotin, avidin, synthetic polymer, polymeric microparticle, nanoparticle, fluorophore, chromophore, radioisotope, macrocyclic complexes of radioisotope, and combinations thereof.

comprising a step of X-rhodamine formation on the aldehyde function of compound of formula II:

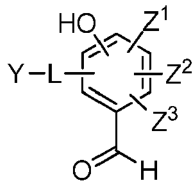

wherein **Z¹, Z²**, **Z³**, **L** and **Y** are as defined above;
to afford compound of formula I.

According to one embodiment, the formation of the X-rhodamine is performed according to methods well-known by those skilled in the art. According to a preferred embodiment, the aldehyde function of compound of formula II is allowed to react in presence of 8-hydroxyjulolidine and para toluene sulfonic acid and then further in presence of chloranil. According to an embodiment, the aldehyde function of compound of formula II is allowed to react with 2 equivalents of 8-hydroxyjulolidine and a catalytic amount of para toluene sulfonic acid, preferably 0.1 equivalent. Preferably, the reaction is conducted at room temperature. Preferably, the solvent is propionic acid. Preferably the reaction is conducted for a period of time of about 12 hours. According to one embodiment, a solution of chloranil is then added, preferably a solution in dichloromethane. Preferably, 1 equivalent of chloranil is added.

According to one embodiment, the process of the invention further comprises one or more subsequent of modification of Y substituent.

In one embodiment, the modification of Y substituent is performed in the case wherein Y is a reactive function (Y'), in order to introduce a bioactive group (Y"). In this embodiment, the step of modification of Y substituent comprises reacting the reactive function Y' with a bioactive group, to link the bioactive group to the compound of the invention, leading to Y". Consequently, when Y represents a bioactive group, it is implicit that the bioactive group may comprise a linking moiety, such as for example an ester bound, an amide bound or a triazolo group (click chemistry), to be linked to linker L.

**Use of the compounds: pH determination**

The present invention further relates to the use of the compounds of the invention as pH probes. Especially, the compounds of the invention are useful for the determination of pH in a sample of interest. In an embodiment, the present invention relates to a method of determining intracellular pH comprising the use of the compounds of the invention.
The pH probes of the present invention can be used to determine the pH of living cells or cell compartments, a change in pH to the local environment caused by a cell as well as directly and indirectly detect specific cellular events associated with a change in pH.
By "a cell compartment" it is referred to one of the many organelles present in the cell cytoplasm.
The pH of a cell or cell compartment can be measured by introducing a probe or a functionalized probe into a cell or cell compartment, irradiating the probe with a suitable light source, and observing the intensity of fluorescence of the probe. The observed fluorescence intensity can then be used to determine pH. For instance, the observed fluorescence may be compared to a known standard, for example a calibration curve of fluorescence intensity versus pH, or to fluorescence intensity measurements indicative of the total probe present.
According to one embodiment, any conventional fluorimetric equipment can be used to irradiate the sample, and to measure the resulting fluorescent response.
According to an embodiment, the sample comprises live cells, intracellular fluids, extracellular fluids, biological fluids, sera, biological fermentation media, environmental sample, industrial samples, proteins, peptides, buffer solutions, biological fluids or chemical reactors, blood cells, immune cells, cultured cells, muscle tissue, neurons, extracellular vesicles; vascular tissue, blood fluids, saliva, urine, water, soil, waste water, sea water; pharmaceuticals, foodstuffs or beverages.
In one embodiment of the invention, the sample is immobilized on a solid or semi-solid support, preferably on a polymeric membrane, within a polymeric gel, on a microparticle, on a microarray, on a silicon chip, on a glass slide, on a microwell plate, or on a microfluidic chip.
The present invention also provides a method for determining the pH of a sample, the method comprising:

(a) contacting the sample with a pH probe according to the invention, to form a contacted sample;
(b) incubating the contacted sample for an appropriate amount of time to form an incubated sample;
(c) illuminating the incubated sample at an exciting wavelength that generates a fluorescent response to form an illuminated sample; and
(d) detecting fluorescent emissions from the illuminated sample;

wherein the fluorescent emissions are used to determine the pH of the sample.
According to an embodiment, the present invention provides a method for monitoring the pH inside a live cell, the method comprising:

(a) contacting the cell with a pH probe according to the invention to form a contacted cell;
(b) incubating the contacted cell for an appropriate amount of time for the compound to enter the cell to form a labeled cell;
(c) illuminating the labeled cell at an exciting wavelength that generates a fluorescent response to form an illuminated cell; and
(d) detecting fluorescent emissions from the illuminated cell;

wherein the fluorescent emissions are used to monitor the pH inside the cell.
When the pH probe of the invention is under a functionalized form, it advantageously enables targeting specific compartments inside the cell.
A change in the pH inside the cell may correspond to a cellular process. Therefore, according to an embodiment, the present invention provides a method for detecting a pH related intracellular process, the method comprising:

(a) contacting a pH probe according to the invention with a cell to form a contacted cell;
(b) incubating the contacted cell for an appropriate amount of time to form an incubated solution;
(c) illuminating the incubated solution at an exciting wavelength that generates a fluorescent response to form an illuminated solution; and
(d) detecting fluorescent emissions from the illuminated solution;

wherein increased fluorescent emissions indicates activation of the intracellular process.
According to an embodiment, the method for monitoring the pH inside a live cell further and/or the method for detecting a pH related intracellular process further comprises:

- stimulating the cell;
- monitoring changes in the intensity of the fluorescent response from the probe; and
- correlating the changes in fluorescence intensity with changes in intracellular pH.

According to one embodiment, cells of potential interest for detecting intracellular pH, but are not limited to, primary culture of mammalian cells, cells dissociated from mammalian tissues. Cell types may include white blood cell, hepatocytes, pancreatic beta cells, neurons, smooth muscle cells, intestinal epithelial cells, cardiac myocytes, glial cells, and the like.

In one embodiment, the sample or medium in which the pH probe is present is illuminated with a wavelength of light selected to give a detectable optical response, and observed with a means for detecting the optical response. Equipment that is useful for illuminating the compounds of the invention includes, but is not limited to, handheld ultraviolet lamps, mercury arc lamps, xenon lamps, lasers and laser diodes. These illumination sources are usually optically integrated into laser scanners, fluorescence microplate readers or standard or microfluorometers.

According to an embodiment, the compound of the invention is functionalized with a fluorophore, preferably a blue probe, a green probe, a far red (>625 nm) probe and an IR(>670 nm) probe . Such a functionalized compound enables bimodal imaging. In an embodiment, the fluorescence of the fluorophore coupled to the compound of the invention does not depend from the pH. In this case, ratiometric studies may be conducted using the fluorophore-functionalized compound of the invention.

In an embodiment, the compound of the invention is functionalized with a radioactive Positron Emission Tomography PET-probe, such as for example $^{18}F$, DOTA or NOTA $^{68}Ga$ complexes, enabling PET-scan together with pH imaging. DOTA refers to 1,4,7,10-tetraazacyclododecane-1,4,7,10-tetraacetic acid, and NOTA represents NOTA-1,4,7-triazacyclononane-1,4,7-triacetic acid.

In a specific embodiment, the pH probe of formula I of the invention, when having R being $N_3$, may be co-administered with modified biomacromolecules to operate intracellular click reaction, as suggested by Takei et al. (Takei et al., ChemComm, 2013, 49, 7313-7315). Especially, the compound of the invention may be administered with a bibenzylcyclooctyl-modified biomacromolecule. The click reaction between the azide function and the alkyne moiety of the modified biomacromolecule aims at retaining the pH probe in the cytosol of tested cells.

<u>Kit</u>

The present invention further relates to a kit for determining the pH of a sample, comprising a compound according to the invention. The kit of the invention may comprise a compound of the invention either present as a pure compound, in a suitable carrier composition, or dissolved in an appropriate stock solution. The kit may further comprise instructions for the use of the pH probe of the invention. The kit may further comprise one or more additional components, such as an additional detection reagent. According to an embodiment, the kit further comprises calibration standards. In one embodiment, the kit of the invention may further comprise a positive control, not dependent from pH, to determine the maximum of fluorescence which may be expected.

According to one embodiment, the pH probe of the invention may be present in the kit associated with a surface, such as for example a chip, microplate well, or other solid or semi-solid matrix.

According to one embodiment, the kit further comprises buffers and/or stabilizers. According to another embodiment, the kit further comprises indicator solutions, blotters, culture media, cuvettes, and the like. In another exemplary embodiment, the kit comprises indicator cartridges (where a kit component is bound to a solid support) for use in an automated detector.

## BRIEF DESCRIPTION OF THE DRAWINGS

**Figure 1** is a drawing schematically representing the red-emitting pH probe of the invention.

**Figure 2** is a graph representing the dependence of absorption on pH in a MOPS buffered aqueous solution for compound **I-1.**

**Figure 3** is a graph representing the dependence of fluorescence intensity on pH in a MOPS buffered aqueous solution after excitation at 535 nm, for compound **I-1.**

**Figure 4A-4C** are graphs representing the dependence of fluorescence enhancement on pH for compounds **I-1** (4A), **I-3** (4B) and **I-4** (4C).

**Figure 5** is a graph representing fluorescence intensities of compound **I-1** (5 μM) in presence of a range of metal ions in a MOPS buffer (MOPS 30 mM, KCl 100 mM, pH 7.2). The concentration of cations is $10^{-3}$ M.

**Figure 6** is a graph representing CCK-8 test results for compound **I-1** indicating cytotoxicity. The probe was incubated with Chinese hamster ovary cells (CHO-K1) for 2 hours at 37°C.

**Figure 7** is a dose response diagram for F98 cells incubated with pH probe **I-1.**

**Figure 8** is a graph representing the fluorescence spectrum for the dual-fluorophore **I-5** (5 μM) measured in an aqueous MOPS buffered solution/MeOH, 91/9.

**Figure 9** is a plot of $I_{\sim612}/I_{439}$ versus pH at excitation wavelength 405 nm for compound **I-5.** $I_{\sim612}$ and $I_{439}$ are the respective fluorescence intensities of the system at ~612nm corresponding to the X-rhodamine red-emission and at 439nm corresponding to the acridone blue-emission.

## EXAMPLES

The present invention is further illustrated by the following examples.

## I. SYNTHESIS

Abbreviations

ACN: acetonitrile
AscNa: sodium ascorbate
Cyc: cyclohexane
DCM: dichloromethane
DMF: dimethylformamide
EDTA: ethylenediaminetetraacetic acid
NMR: nuclear magnetic resonance
PTSA: p-toluenesulfonic acid
TFA: trifluoroacetic acid
THF: tetrahydrofurane

Material

All the solvents were of analytical grade. Chemicals were purchased from commercial sources. The salts used in stock solutions of metal ions were $CaCl_2{\cdot}2H_2O$, $CuCl_2{\cdot}2H_2O$, $FeCl_3{\cdot}6H_2O$, $MgCl_2{\cdot}6H_2O$, $MnCl_2{\cdot}4H_2O$, $ZnBr_2$. $^1$H-NMR and $^{13}$C-NMR were measured on a Bruker avance III-300 MHz spectrometer with chemical shifts reported in ppm (TMS as internal standard). Mass spectra were measured on a Focus GC / DSQ II spectrometer (ThermoScientific) for IC and an API 3000 spectrometer (Applied Biosystems, PE Sciex) for ES. All pH measurements were made with a Mettler Toledo pH-Meter. Fluorescence spectra were recorded on a JASCO FP-8300 spectrofluorometer. Absorption spectra were determined on a VARIAN CARY 300 Bio UV-Visible spectrophotometer. All measurements were done at a set temperature of 25°C. The purity of the dyes were checked by RP-HPLC C-18, eluant: ACN 0.1% TFA/Water 0.1 % TFA, method: 20/80 to 100/0 within 20 min. then 100/0 for 10 min. detection at $\lambda_{abs}$= 254 nm.

### *I.1. General X-rhodamine synthesis*

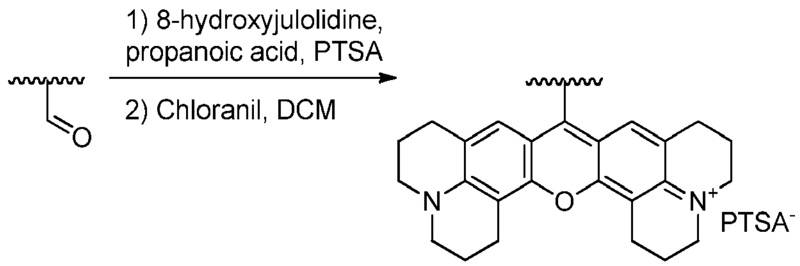


To a solution of aldehyde (1eq) in propanoic acid was added 8-hydroxyjulolidine (2 eq) and PTSA (1 eq). The solution was protected from light and stirred at room temperature overnight. To the brown mixture was added a solution of chloranil (1 eq) in DCM./MeOH. The reaction turned dark and was allowed to stir overnight at room temperature. The dark purple solution was evaporated to dryness. The crude was purified by column chromatography on silica gel (DCM/MeOH, initially 98/2 reaching 90/10 over 30 min.) to obtain the X-Rhodamine product as a purple solid.

***I.2. Aldehyde starting materials***

A mixture of 5-formyl-2-hydroxybenzoic acid (2 g, 12.04 mmol, 1 eq) and $SOCl_2$ (20 ml, 274.19 mmol, 23 eq) was stirred and heated to 85°C overnight. The mixture was concentrated to dryness and left overnight under vacuum to yield the aromatic acyl chloride product 1 as a white solid (2.22 g, 12.02 mmol, 99%).

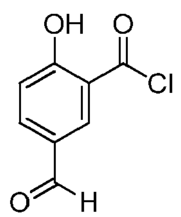

To a solution of the previously synthesized aromatic acyl chloride **1** (2.22 g, 12.03 mmol, 1 eq) in DCM (60 ml) was added a solution of tert-butyl-1-piperazinecarboxylate (2.70, 14.43 mmol, 1.2 eq) in DCM (30ml), triethylamine (5.1 ml, 36.9 mmol, 3 eq) and THF (20 ml). The reaction mixture was left stirring overnight and concentrated. The crude product was treated with DCM/HCl (1M) and a precipitate formed. The solid was filtered off and the filtrate further extracted with DCM. The combined organic phases were washed with $NaHCO_3$, dried over $MgSO_4$ and evaporated to dryness. The solid was purified by column chromatography on silica gel (DCM/MeOH, 97/3) to yield the product **2** as a yellow solid (1.31 g, 3.91 mmol, 33%). **$^1$H NMR** (300 MHz, Chloroform-d) δ 10.45 (s, 1H, O<u>H</u>), 9.79 (s, 1H, C<u>H</u>O), 7.80 (dd, $J_{a\text{-}b}$ = 8.1 Hz , $J_{c\text{-}b}$ = 1.8 Hz, 1H, $H_b$), 7.75 (d, $J_{b\text{-}c}$ = 2.1 Hz, 1H, $H_c$), 7.07 (d, $J_{a\text{-}b}$ = 8.5 Hz, 1H, $H_a$), 3.66 - 3.38 (m, 8H, $(CH_2)_4$), 1.42 (s, 9H, $(Me)_3$). **$^{13}$C NMR** (75 MHz, $CDCl_3$) δ 189.91 (<u>C</u>HO), 170.09 (<u>C</u>ON), 164.49 ($COO^tBu$), 154.46 (C Ar), 134.74 (C Ar), 130.26 (C Ar), 127.97 ($C_b$), 118.90 ($C_c$), 116.96 ($C_a$), 80.72 (<u>C</u>$(Me)_3$), 45.71 ($CH_2$), 43.56 ($CH_2$), 28.38 (Me).

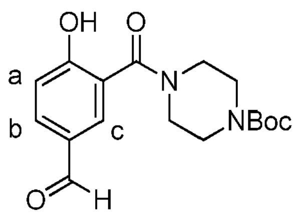

To a solution of **2** (200 mg, 0.60 mmol, 1 eq) in DCM (10 ml) was added TFA (1.25 ml, 2.98 mmol, 5 eq). The solution was stirred at 0°C for 1h before being left under ultrasound at room temperature for a further 1h 30min. The reaction mixture was poured into diethyl ether (125 ml) to form a sticky, white precipitate. The solid was filtered, redissolved in MeOH and evaporated to dryness to yield the product **3** a white solid (90 mg, 0.26 mmol, 43%).

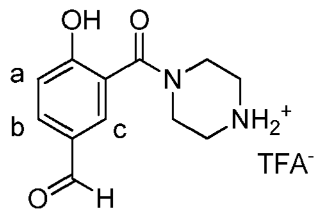

To a solution of **3** (200 mg, 0.26 mmol, 1 eq) and triethylamine (0.18 ml, 1.29 mmol, 5 eq) in DCM (10ml) at 0°C was added propargyl chloroformate (0.05 ml, 0.52 mmol, 2 eq). After stirring for 20 min. the solution was concentrated to dryness and dissolved in MeOH. $K_2CO_3$ (0.07g, 0.51 mmol, 2 eq) was added to the solution, MeOH was evaporated and the solid extracted with EtOAc/HCl. The combined organic layers were dried over $MgSO_4$ and the EtOAc evaporated to yield the product **4a** (0.17g, 5.36 mmol, 94%). **$^1$H NMR** (300 MHz, Chloroform-*d*) δ 9.73 (s, 1H, CHO), 9.04 (s, 1H, OH), 7.74 - 7.70 (m,1H, $H_c$), 7.25 - 7.17 (m, 1H, $H_b$), 6.98 - 6.95 (d, $J_{a-b}$ = 12Hz, 1H, $H_a$), 4.66 (d, *J* = 2.5 Hz, 2H, $OCH_2$), 3.79 - 3.32 (m, 8H, H-pip), 2.45 (t, *J* = 2.4 Hz, 1H, H-alkyne).

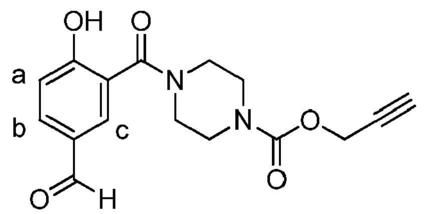

To a solution of 3 (156mg, 0.45 mmol, 1 eq) in DCM (5 ml) was added triethylamine (0.40 ml, 2.69 mmol, 6 eq) and propargyl bromide (80 % in toluene) (0.20 ml, 1.34 mmol, 3 eq). The mixture was stirred for 24h before a further 3 eq propargyl bromide and 6 eq triethylamine were added. The mixture was stirred for a further 15h and the concentrated to dryness. The solid was purified by column chromatography on silica gel (DCM/MeOH, 95/5) to yield the product **4b** (101 mg, 0.37 mmol, 82%). **$^1$H NMR** (300 MHz, Chloroform-*d*) δ 9.88 (s, 1H, CHO), 7.91 - 7.81 (m, 2H, $H_b$, $H_c$), 7.13 (d, $J_{a-b}$ = 8.4 Hz, 1H, $H_a$), 3.82 (t, *J* = 5.0 Hz, 4H, $CON(CH_2)_2$), 3.40 (d, J = 2.5 Hz, 2H, $NCH_2$), 2.68 (t, J = 5.0 Hz, 4H, $N(CH_2)_2$, 2.33 (s, 1H, H-alkyne). **$^{13}$C NMR** (75 MHz, $CDCl_3$) δ 190.07 (CHO), 169.57 (CON), 164.37 (ArCOH), 134.46 (C Ar), 130.43 (C Ar), 127.88 (C Ar), 118.76 (C Ar), 117.38 (C Ar), 77.77 ($CH_2CCH$), 74.10 ($CH_2CCH$), 51.62 (C-pip), 46.73 ($CH_2$), 45.57.

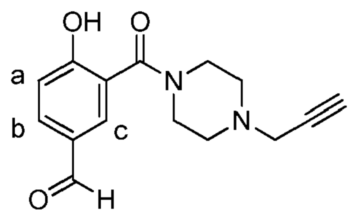

To a solution of the previously synthesized aromatic acyl chloride 1 (2.77 g, 15 mmol, 1 eq) in dry DCM (25 ml) was added 2-propyn-1-amine (1.5 ml, 22.5 mmol, 1.5 eq) and triethylamine (8.4 ml, 60 mmol, 4 eq). The solution was stirred for 15h and extracted with DCM/HCl with formation of a precipitate, which was subsequently filtered off. The combined organic phases together with the filtered solid were purified by column chromatography on silica gel (Cyc/EtOAc, 7/3) via solid deposition to yield the product **4c** (0.33 g, 1.6 mmol, 11%). **$^1$H NMR** (300 MHz, DMSO-$d_6$) δ 13.09 (s, 1H, OH), 9.87 (s, 1H, CHO), 9.39 (t, J = 5.7 Hz, 1H, NH), 8.52 - 8.44 (m, 1H, $H_c$), 7.96 (dd, $J_{a-b}$ = 8.4 Hz, $J_{b-c}$ = 2.0 Hz, 1H, $H_b$), 7.13 (d, $J_{a-b}$ = 8.6 Hz, 1H, $H_a$), 4.14 (dd, J = 5.6, 2.5 Hz, 2H, $NCH_2$), 3.33 - 3.15 (m, 1H, H-alkyne). **$^{13}$C NMR** (75 MHz, DMSO) δ 191.28 (CHO), 167.71 (CON), 164.82 (ArCOH), 134.81 (C Ar), 132.13 (C Ar), 128.56 (C Ar), 118.70 (C Ar), 116.58 (C Ar), 81.00 (CCH), 73.86 (CCH), 29.00 ($CH_2$).

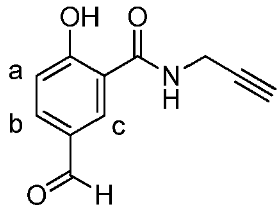

To a solution of 3-azidopropylamine (1.5 g, 15 mmol, 1 eq) and triethylamine (8.4 ml, 60 mmol, 4 eq) under argon was added the previously synthesized aromatic acyl chloride **1** (2.7 g, 15 mmol, 1 eq) in dry DCM (25 ml) to form a yellow homogeneous solution that was left stirring overnight. The reaction was extracted with DCM/HCl and the combined organic phases dried over $MgSO_4$.. The crude product was purified by column chromatography (Cyclohexane/EtOAc, 75/25) via a solid deposition to yield the pure product as a **4d** (0.52 g, 2.1 mmol, 14%). **$^1$H NMR** (300 MHz, Chloroform-*d*) $\delta$ 13.08 (s, 1H, OH), 9.81 (s, 1H, CHO), 7.93 (d, $J_{c-b}$ = 1.9 Hz, 1H, $H_c$), 7.84 (dd, $J_{b-a}$ = 8.6 Hz, $J_{c-b}$ = 2.0 Hz, 1H, $H_b$), 7.05 (d, $J_{a-b}$ = 8.6 Hz, 1H, $H_a$), 6.76 (s, 1H, NH), 3.53 (dt, *J* = 6.6 Hz, *J* = 6.0 Hz, 2H, $NHCH_2$), 3.47 - 3.38 (t, J = 6.3 Hz, 2H, $CH_2N_3$), 1.88 (p, J = 6.6 Hz, 2H, $NHCH_2CH_2$). **MS (CI):** calcd for $C_{11}H_{12}N_4O_3$ $[M+NH_4]^+$, 248.09; found 249.13.

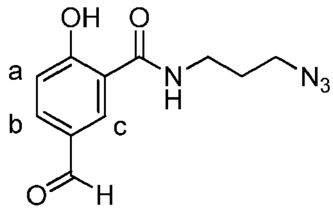

### *I.3. X-Rhodamine construction*

| | |
|---|---|
| 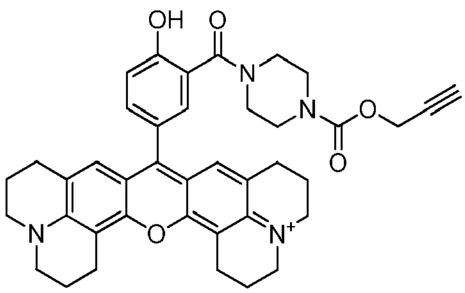 | From starting aldehyde **4a** the X-rhodamine **I-3** was synthesised via the method outlined in general directions (46 mg, 0.07 mmol, 11%). **MS (ES+):** calcd for $C_{40}H_{41}N_4O_5^+$ $[M]^+$, 655.33; found 656.7. |
| 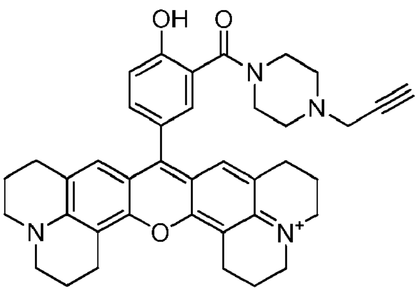 | From starting aldehyde **4b** the X-rhodamine **I-4** was synthesised via the method outlined in general directions (82.3 mg, 0.11 mmol, 28%). **MS (ES+):** calcd for $C_{39}H_{41}N_4O_3^+$ $[M]^+$, 613.32; found 613.6. |

(continued)

| | |
|---|---|
| 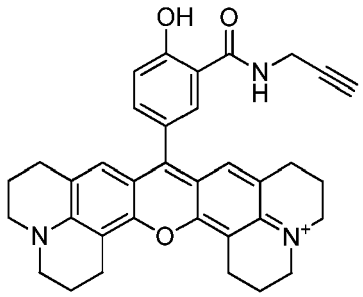 | From starting aldehyde **4c** the X-rhodamine **I-2** was synthesised via the method outlined in general directions (37.2 mg, 0.05 mmol, 4%). |
| 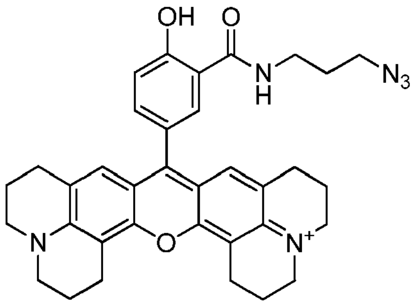 | From starting aldehyde **4d** the X-rhodamine **I-1** was synthesised via the method outlined in general directions (11%). **MS (ES+):** calcd for $C_{35}H_{37}N_6O_3^+$ [M]$^+$, 589.29; found 589.6. |

### *I.4. Ratiometric Probe*

<u>Blue Fluorophore</u>

To a solution of 9(10H)-acridanone (0.50 g, 2.56 mmol, 1 eq) in DMF (15ml) was added NaI (0.77 g, 5.12 mmol, 2 eq) and 5-chloro-1-pentyne (0.54 ml, 5.12 mmol, 2 eq). NaH 60% w/w (0.12 g, 3.07 mmol, 1.2 eq) was added. Once bubbling/release of $H_2$ had stopped the solution was heated to 50°C for 4h, cooled to room temperature. DMF was evaporated and the solid extracted 3 times with DCM/$H_2O$ upon which a precipitate formed and was filtered off. The combined organic phases were dried over $MgSO_4$. The organic phases were combined with the filtered precipitate and purified by column chromatography (Cyclohexane/EtOAc, 7/3) via a solid deposition. The solid was recrystallised from EtOH to yield the product 5 as a yellow crystalline solid (34.1 mg, 1.3 mmol, 51%). **$^1$H NMR** (300 MHz, Chloroform-*d*) $\delta$ 8.52 (dd, $J_{c-d}$ = 8.0 Hz, $J_{b-c}$ = 1.7 Hz, 2H, $H_c$), 7.67 (dd, $J_{a-b}$ = 9.2, 4.0 Hz, 2H, $H_b$), 7.52 (d, $J$ = 8.7 Hz, 2H, $H_a$), 7.23 (dd, $J_{d-c}$ = 7.9 Hz, $J_{d-b}$ =1.2 Hz, 2H, $H_d$), 4.51 - 4.42 (t, $J$ = 12 Hz, 2H, $\underline{NCH_2}$), 2.41 (td, $J$ = 6.6, 2.7 Hz, 2H, $NCH_2CH_2\underline{CH_2}$), 2.14 (t, $J$ = 2.6 Hz, 1H, H-alkyne), 2.12 - 2.02 (m, 2H, $NCH_2\underline{CH_2}$). **$^{13}$C NMR** (75 MHz, $CDCl_3$) $\delta$ 177.91 ($\underline{C}O$), 141.78 (CAr), 134.09 (CAr), 128.10 (CAr), 122.42 (CAr), 121.44 (CAr), 114.44 (CAr), 82.52 ($\underline{C}CH_2$), 70.25 ($C\underline{C}H_2$), 44.90 ($N\underline{C}H_2$), 25.34 ($NCH_2\underline{C}H_2$), 16.06 ($NCH_2CH_2\underline{C}H_2$)- **MS (CI):** calcd for $C_{18}H_{15}NO$ [M+H]$^+$, 262.12; found 262.09.

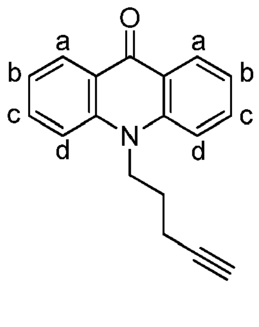

<u>Aldehyde intermediate material</u>

To a solution of **4d** (221 mg, 0.89 mmol, 1 eq) in dioxane (10 ml) was added 5 (513 mg, 1.34 mmol, 1.5 eq) forming a homogeneous yellow solution. $CuSO_4.5H_2O$ (222 mg, 0.89 mmol, 1 eq) and Na ascorbate (176 mg, 0.89 mmol, 1 eq) were mixed in water (1 ml) and added to the solution. The solution was stirred overnight. The solution was evaporated to dryness and the solid extracted with DCM/EDTA (0.1M). The combined organic phases were washed

twice with EDTA (0.1M), dried over $MgSO_4$ and filtered. The crude product was purified by column chromatography (EtOAc 100%) to yield the product 6 (50 mg, 0.10 mmol, 11%). **$^1$H NMR** (300 MHz, Chloroform-*d*) δ 13.26 (s, 1H, OH), 9.59 (s, 1H, CHO), 8.40 (dd, J = 8.1 Hz, J = 1.8 Hz, 2H, $H_1$), 8.23 (d, J = 1.8 Hz, 1H, $H_c$), 7.95 (s, 1H, NH), 7.71 (dd, J = 8.7 Hz, J = 1.8 Hz 1H, $H_b$), 7.59 (dd, J 9.0 = Hz, J = 1.5 Hz, 2H, $H_m$), 7.47 (s, 1H, triazole), 7.39 (d, J = 8.7 Hz, 2H, $H_n$), 7.15 (dd, *J* = 8.4 Hz, J = 0.9 Hz, 2H, $H_k$), 6.92 (d, *J* = 8.6 Hz, 1H, $H_a$), 4.42 (t, *J* = 6.3 Hz, 2H, $CH_2$N-triazole), 4.33 (t, J = 8.1 Hz, 2H, $CH_2NR_2$), 3.46 (q, *J* = 6.2 Hz, 2H, $NHCH_2$), 2.84 (t, J = 7.2 Hz, 2H, $CH_2$C-triazole), 2.21 (m, 4H, $2CH_2CH_2CH_2$). **$^{13}$C NMR** (75 MHz, $CDCl_3$) δ 190.17 (CHO), 177.98 (C=O acridone), 169.82 (CON) 166.84 (ArCOH), 162.66, 146.00, 141.61, 134.86, 134.24, 129.77, 127.86, 127.66, 122.09 (CH triazole), 121.52, 119.13, 114.80, 114.55, 77.49, 77.27, 77.07, 76.64, 48.34, 45.39, 37.02, 36.58, 31.50, 30.20, 29.60, 26.14, 22.57. MS (CI): calcd for $C_{29}H_{27}N_5O_4$ $[M+H]^+$, 510.21; found 510.5.

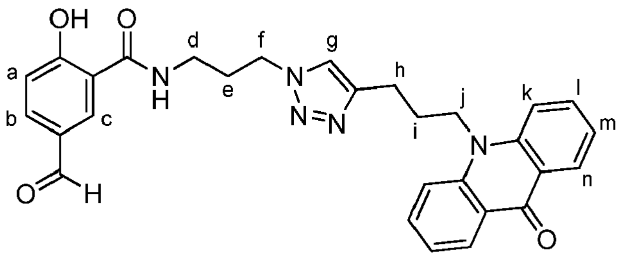

X-Rhodamine construction

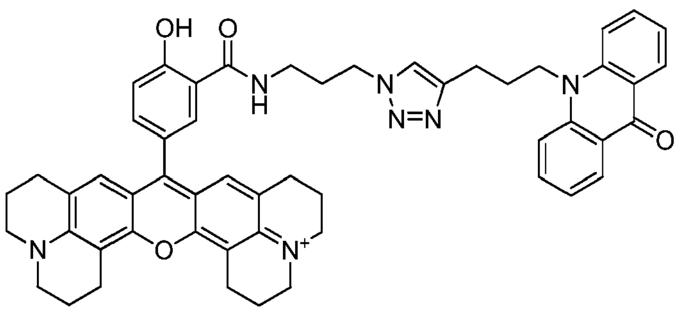

From starting aldehyde 6 the product **I-5** was synthesised via the method outlined in general directions (8.9 mg, 8.7 μM, 9%). MS (ES+): calcd for $C_{53}H_{52}N_7O_4^+$ $[M]^+$, 850.41; found 850.7.

## II. OPTICAL and pH PROPERTIES

### *II.1.* ***Methods***

***Dynamic range***. The term dynamic range refers to the maximal value of equation 1 where I is the observed fluorescence intensity and $I_{min}$ is the minimum fluorescence intensity observed for that probe. The larger the dynamic range the better as it indicates good differentiation between "on" and "off' states.

$$Dynamic\ Range = \frac{(I - I_{min})}{I_{min}} \qquad \text{(Equation 1)}$$

***Quantum yield calculations.*** The fluorescence quantum yields (Φ) were calculated from the gradient of integrated fluorescence intensity (545 to 700 nm) versus optical density. Rhodamine 101 (Φ = 1.0 in absolute ethanol) was used as a reference standard (ref) with the excitation wavelength at 535 nm. Equation 2 was used to determine the Φ values.

$$\Phi = \Phi_{ref} \frac{grad.}{grad._{ref}} \cdot \frac{\eta^2}{\eta_{ref}^2} \qquad \text{(Equation 2)}$$

Φ = quantum yield
η = refractive index of the solvent system (η ($H_2O$) = 1.33, $\eta_{ref}$ (EtOH) = 1.36) grad. = gradient of the curve

$\Phi_{ON}$ (pH = 4) and ΦOFF (pH = 10) were calculated for each pH probe in MOPS 30 mM, KCl 100 mM.

***Molar extinction coefficient calculations.*** Extinction coefficients (ε) were calculated using the Beer Lambert law (equation 3) plotting optical density/absorption versus concentration for the pH probes. The gradient of the plot is equal to ε.

$$A = \varepsilon cl \qquad \text{(Equation 3)}$$

A = optical density/absorption
ε = extinction coefficient = gradient of the plot
c = concentration
1= optical path length (1 cm)

***pKa calculations.*** pKa values were calculated by plotting the fluorescence intensity at $\lambda_{em\cdot,max}$ versus the pH. The data was fit to the Hill Equation (equation 4) and the pKa taken as $x_{1/2}$ as per the Hill equation.

$$base + \frac{(\max - base)}{1+\left(\frac{x_{1/2}}{x}\right)^{rate}} \qquad \text{(Equation 4)}$$

base = y value at minimum x-value
max = y value at maximum x-value
rate = rise rate of the curve
$x_{1/2}$ = x value at which y = (base + max)/2.

***II.2. Spectrocopic data and pKa.***

The spectroscopic data for the four "clickable" probes I-1, I-3 and **I-4** are shown below:

| **Compound** | | **$\lambda_{abs}$ (nm)** | **$\lambda_{em}$ (nm)[c]** | **pKa** | **ε ($M^{-1}.cm^{-1}$)** | **Φ** | **$\Phi_{ON}$/ $\Phi_{OFF}$** | **Dynamic Range** |
|---|---|---|---|---|---|---|---|---|
| **I-1** | ON[a] | 580 | 600 | 3.96 | 26742 | 0.254 | 28 | 88 |
| | OFF[b] | 574 | 596 | ±0.03 | 26706 | 0.009 | | |
| **I-3** | ON[a] | 580 | 601 | 7.68 | 72405 | 0.640 | 40 | 86 |
| | OFF[b] | 575 | 602 | ±0.09 | 49298 | 0.016 | | |
| **I-4** | ON[a] | 580 | 601 | 7.64 | 35109 | 0.798 | 35 | 98 |
| | OFF[b] | 574 | 600 | ±0.10 | 20712 | 0.023 | | |

[a] Protonated form: pH 4
[b] Deprotonated form: pH 10
[c] Excitation at 535 nm
$\lambda_{abs}$ refers to the maximum absorption wavelength
$\lambda_{em}$ refers to the maximum emission wavelength

The dependence of absorption on pH for probe I-1 was determined in a MOPS buffered aqueous solution. Results are represented in figure 2.

The dependence of fluorescence intensity on pH for probe I-1 was determined in a MOPS buffered aqueous solution after excitation at 535 nm. Results are represented in figure 3.

The dependence of fluorescence enhancement on pH was determined for probes I-1, I-3 and I-4. Results are represented in figure 4A, 4B, and 4C respectively. Curve fitting was based on a modified Hill equation from which $pK_a$ values were calculated.

## III. BIOLOGICAL STUDIES

### *III.1. Methods*

***Cytotoxicity:*** CHO-K1 cells (20,000 per well) were seeded in 96 multiple well plates for 24 h before the experiment. Cells (40,000 per well) were incubated with the fluorescent compounds in 100 μL DMEM for 2h at 37°C. The incubation medium was replaced by 100μL fresh DMEM containing 10% of cell-counting solution (Dojindo Laboratories) and further incubated for 2h at 37°C. The absorbance was measured at 450 nm (reference at 620 nm) and was directly related to the number of living cells. Experiments were done in triplicate and repeated twice.

***Cell calture for flow cytometry, dose response and cellular imaging:*** F98 and CHO-K1 cell lines (purchased from ATCC) were maintained at 37°C in 5% $CO_2$ in DMEM/F-12 nutrient medium (Invitrogen, Cergy Pontoise, France) supplemented with 2% (v/v) heat-inactivated fetal bovine serum (Invitrogen) and 100 μg/mL streptomycine and 100 units/mL penicillin (Invitrogen).

***Fluorescence Activated Cell Sorting Analyses:*** F98 cells were incubated with various concentrations of pH probes in DMEM/F-12 culture medium without serum at 37 °C for 2h. The cells were washed with PBS to remove excess fluorescent probe and were treated with 0.05% Trypsin-EDTA for 2 min at 37°C to detach the cells from the surface, and centrifuged at 200 g in DMEM/F-12 culture medium before suspension in PBS. Flow cytometry analyses were performed with live cells using an Accuri C6 flow cytometer (BD Biosciences, Le Pont de Claix, France). The data was obtained and analysed using CFlow Sampler (BD Biosciences). Live cells were gated by forward/side scattering for a total of 10,000 events.

***Confocal Microscopy:*** Cell cultures were incubated with the pH probes (in DMEM/F-12 nutrient medium only) for 2h, and washed twice with phosphate-buffered saline. The plasma membrane was stained with 50 μg/mL Alexa 647-conjugated concanavalin A (Invitrogen, Cergy Pontoise, France) for 5 min. Cells were washed once more. Live cells were immediately analyzed by confocal laser scanning microscopy using a Zeiss LSM operating system. Alexa 647 (633 nm) and pH probes (561 nm) were sequentially excited and emission fluorescence was collected.

### *III.2. Metal screening*

It must be ensured that any pH probes are specific to their intended function and are not affected by other biologically important species such as free metal ions. For the probes I-1 to I-4, a metal screening was conducted to check for fluorescence quenching.

The metal ions chosen for the screening are biologically important species: $Zn^{2+}$, $Mg^{2+}$, $Ca^{2+}$, $Fe^{3+}$ and $Mn^{2+}$. Ethylenediaminetetraacetic acid (EDTA) was used as a control as it is an excellent ligand for cationic species and so capture any trace metal ions present in the analytical medium. The fluorescence observed in the EDTA-system can therefore be taken as the fluorescence of the naked probe.

$Cu^{2+}$ is not an overly important biological species, however $Cu^{2+}$ has an incomplete valance d-orbital, with the electronic configuration $[Ar]3d^9$. Therefore, it is a paramagnetic species that can quench the fluorescence. It has already been observed that $Cu^{2+}$ can quench X-rhodamine fluorescence. It was thus included in the metal screening.

The results of the metal screening for compound **I-1** are shown in figure 5. Insignificant fluctuations were observed compared to "naked" EDTA environment for all tested metals, which do not affect the on/off function of the probe.

The same results were obtained for compounds **I-3** and I-4. Therefore, in a cellular environment, probes of the invention will continue to act as pH indicator and not be disrupted by the presence of tested metal ions.

### *III.3. Cytotoxicity*

One of the most important biological test to conduct before the probes can be used in cellular imaging is cytotoxicity. Cytotoxicity causes cell death, hence it is very important for the probes to be tested before use in biological imaging studies.

To determine cytotoxicity a CCK-8 cell counting kit was used. This can be bought commercially and relies upon the reduction of a highly water-soluble tetrazolium salt (WST-8). This compound is slightly yellow in color, however, upon reduction to its corresponding formazan species it becomes bright orange. The optical density of the absorbance corresponding to the orange formazan species, detected at 450nm, can be measured and correlated to the number of living cells. A decrease in optical density corresponds directly to a decrease in the quantity of formazan molecules indicating fewer living cells due to cell death/cytotoxicity. The reduction of WST-8 is achieved by exploiting the redox

reactions of NAD+, a coenzyme found in all living cells. Cellular NAD+ is constantly being reduced to NADH by dehydrogenases as a natural part of a cell's metabolism. The subsequent oxidation of NADH back to NAD+ can be used to drive the reduction of electron mediators such as 1-methoxy PMS. These electron mediators can then be re-oxidised allowing the conversion between WST-8 and its reduced formazan form.

A cytotoxic compound is demonstrated by the use of Triton, a non-ionic detergent that causes total cell death used here at 1% Triton concentrations. This can be compared to a control that exhibits normal levels of cell death giving an optical density that can be used to represent efficient cell function. The results of the cytotoxicity test are shown in figure 6.

In order to aid solubility of the probes a small amount of DMSO was used. A control for 0.5% DMSO was thus performed, which shows almost identical results to that of the free control.

***III.4. Dose response***

Undifferentiated malignant glioma rat cells (F98) were incubated with probe **I-1** for two hours at increasing concentrations and the fluorescence intensity measured. Results are represented in figure 7.

The increase of fluorescence with increasing concentration of probe is evidencing the successful internalization of the pH probe into the cells.

**IV. RATIOMETRIC PROBES**

A key advantage of the functionalizable X-rhodamine probes of the invention is the ability to introduce a second fluorophore and so create a ratiometric system. Probes able to give a qualitative indication of pH, switching sharply on and off at precise pH values, is often sufficient in biological studies where the probe has been selected to match the pH range of interest, hence the importance of creating a catalogue of pKa values. However, ratiometric systems may also be provided to obtain quantitative results.

In order to achieve ratiometric measurements with reference to pH, two sources of fluorescence are needed: one in which the fluorescence is pH dependent and a second that remains constant in all pH environments. This can be achieved in two ways.

The first method uses a single pH-dependent fluorophore that changes its $\lambda_{em}$. upon protonation. The protonated and deprotonated forms emit at different wavelengths and so the ratio between the two can be measured to give a quantitative pH reading.

The second method requires two fluorophores, one of which is pH-dependent and the other that emits at a constant wavelength and intensity irrespective of the pH. By calibrating the ratio between the two fluorescent signals, a precise pH value can be obtained.

This second method was applied to probe **I-1** to form probe **I-5**, by the introduction of a second fluorophore upon click reaction.

Careful consideration must be taken when selecting the second pH-independent fluorophore for use in an X-rhodamine based system. The X-rhodamine **I-1** absorbs light at 580 nm in the red region of the spectrum with a reasonably high molar extinction coefficient. Ideally the $\lambda_{em}$ of the second fluorophore would be much lower than this, towards the blue end of the spectrum, so as to avoid Förster resonance energy transfer (FRET). Additionally, sharp peaks are desirable again to minimize the FRET interaction. This can be judged by the signals full width at half its maximum (FWHM).

In addition to having the correct absorption and emission wavelengths, the chosen second fluorophore must feature a terminal alkyne to make it "clickable" with the azide-containing pH probe **I-1.** The acridone **5** was selected, having the following optical properties (measured in $CH_3CN/H_2O$):

| **Fluorophore** | $\lambda_{abs}$ **(nm)** | $\lambda_{em}$ **(nm)** | **FWHM** |
|---|---|---|---|
| 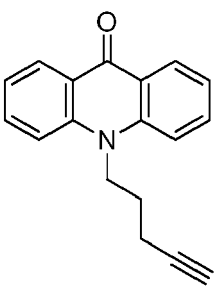 **5** | 408 | 425 | 49 |

This compound fits into the blue region of the spectrum and has a small FWHM value.

The final ratiometric pH probe **I-5** was obtained as described above.

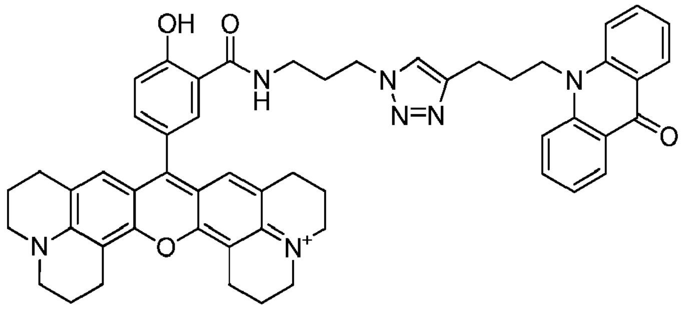

**I-5**

Fluorescence properties in function of pH were determined for compound I-5 and results are reported in figure 8. The peak centred at 439 nm corresponding to emission from the acridone fluorophore and the peak centred at 612 nm to that of the X-rhodamine. The blue acridone emission is not dependent on pH while the red X-rhodamine emission is dependent on pH.

In order to visualize the intensity ratio between the two emission signals a plot of $I_{\sim612}/I_{439}$ versus pH was plotted (figure 9). $I_{\sim612}$ and $I_{439}$ are the respective fluorescence intensities of the system at ~612 nm corresponding to the X-rhodamine red-emission and at 439 nm corresponding to the acridone blue-emission. The plot clearly shows a distinct increase in red emission relative to blue-emission as the system was acidified, shown by a four-fold increase in the $I_{\sim612}/I_{439}$ ratio.

## Claims

1. A compound of formula I:

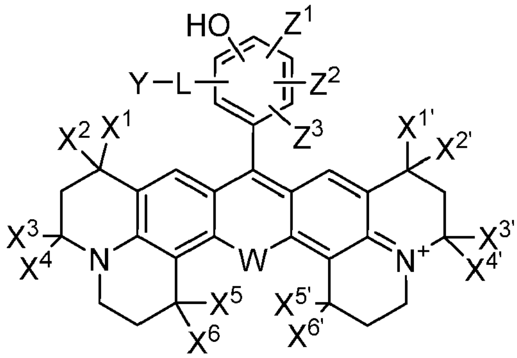

and salts thereof, wherein:

**W** represents O, NH, N(alkyl), Si(alkyl)$_2$, $CH_2$, CH(alkyl), C(alkyl)$_2$; preferably **W** represents O;
**$X^1$** and **$X^{1'}$** represent both H, both Me or both $-CH_2-SO_3H$;
**$X^2$** and **$X^{2'}$** represent both H or both Me;
**$X^3$** and **$X^{3'}$** represent both H or both Me;
**$X^4$** and **$X^{4'}$** represent both H or both Me;
**$X^5$** and **$X^{5'}$** represent both H or both Me;
**$X^6$** and **$X^{6'}$** represent both H or both Me;
**$Z^1$**, **$Z^2$** and **$Z^3$**, represent each independently H, electron withdrawing group or electron donating group, preferably **$Z^1$**, **$Z^2$** and **$Z^3$**, represent each independently H, alkyl, aryl, amino, alkylamino, dialkylamino, thiol, alkylthio, hydroxy, alkoxy, acylamino, (carboxyl ester)oxy, halo, $NO_2$, CN, $-SO_3H$, -COOH, ester, aldehyde, ketone;
**L** represents a single bound or a linker, preferably the linker is selected from aminocarbonyl, alkylaminocarbonyl, aminothiocarbonyl, alkyloxycarbonyl, alkyl, aryl, alkylaryl, arylalkyl, alkoxy, polyethylene glycol (PEG), polypropylene glycol (PPG), peptide or a combination thereof; optionally interrupted or terminated by -O-, -S-, -$SO_2$ or a combination thereof; optionally additionally comprising a residue of a reactive group through which L is bounded to **Y;**

**Y** represents:

- a reactive group selected from the group comprising $N_3$, amino, alkylamino, COOH, amide, maleimide, alkyne, SH, OH, ester, activated ester, activated carboxylic acid, halo, nitro, nitrile, isonitriles, acrylamide, aldehyde, ketone, acetals, ketals, anhydride, glutaric anhydride, succinic anhydride, maleic anhydride, thiocyanate, isothiocyanate, isocyanate, hydrazide, hydrazines, hydrazones, ethers, oxides, cyanates, diazo, diazonium, sulfides, disulfides, sulfoxides, sulfones, sulfonic acids, sulfinic acids, sulfates, sulfenic acids, amidines, imides, imidates, nitrones, hydroxylamines, oximes, hydroxamic acids, thiohydroxamic acids, allenes, ortho esters, sulfites, enamines, ynamines, ureas, pseudoureas, semicarbazides, carbodiimides, carbamates, imines;
- a bioactive group selected from amino acid, peptide, protein, antibody, enzyme, polysaccharide, dextran, benzylguanine, lipid, lipid assembly, fatty acid, nucleoside, nucleotide, oligonucleotide, hapten, aptamer, ligand, substrate, biotin, avidin, synthetic polymer, polymeric microparticle, nanoparticle, fluorophore, chromophore, radioisotope, macrocyclic complexes of radioisotope, and combinations thereof.

**2.** The compound according to claim **1,** wherein:

**L** represents a linker selected from aminocarbonyl, alkylaminocarbonyl, aminothiocarbonyl, alkyloxycarbonyl, alkyl, aryl, alkylaryl, arylalkyl, alkoxy, polyethylene glycol (PEG), polypropylene glycol (PPG), peptide or a combination thereof; optionally interrupted or terminated by -O-, -S-, -$SO_2$ or a combination thereof; and

**Y** represents a reactive group selected from the group comprising $N_3$, amino, alkylamino, COOH, amide, maleimide, alkyne, SH, OH, ester, activated ester, activated carboxylic acid, halo, nitro, nitrile, isonitriles, acrylamide, aldehyde, ketone, acetals, ketals, anhydride, glutaric anhydride, succinic anhydride, maleic anhydride, thiocyanate, isothiocyanate, isocyanate, hydrazide, hydrazines, hydrazones, ethers, oxides, cyanates, diazo, diazonium, sulfides, disulfides, sulfoxides, sulfones, sulfonic acids, sulfinic acids, sulfates, sulfenic acids, amidines, imides, imidates, nitrones, hydroxylamines, oximes, hydroxamic acids, thiohydroxamic acids, allenes, ortho esters, sulfites, enamines, ynamines, ureas, pseudoureas, semicarbazides, carbodiimides, carbamates, imines.

**3.** The compound according to claim **1,** wherein:

**L** represents a linker selected from aminocarbonyl, alkylaminocarbonyl, aminothiocarbonyl, alkyloxycarbonyl, alkyl, aryl, alkylaryl, arylalkyl, alkoxy, polyethylene glycol (PEG), polypropylene glycol (PPG), peptide; or a combination thereof; optionally interrupted or terminated by -O-, -S-, -$SO_2$ or a combination thereof; optionally additionally comprising a residue of a reactive group through which L is bounded to **Y;** and

Y represents a bioactive group selected from amino acid, peptide, protein, antibody, enzyme, polysaccharide, dextran, benzylguanine, lipid, lipid assembly, fatty acid, nucleoside, nucleotide, oligonucleotide, hapten, aptamer, ligand, substrate, biotin, avidin, synthetic polymer, polymeric microparticle, nanoparticle, fluorophore, chromophore, radioisotope, macrocyclic complexes of radioisotope, and combinations thereof.

**4.** The compound according to anyone of claims **1** to **3,** of formula Ia:

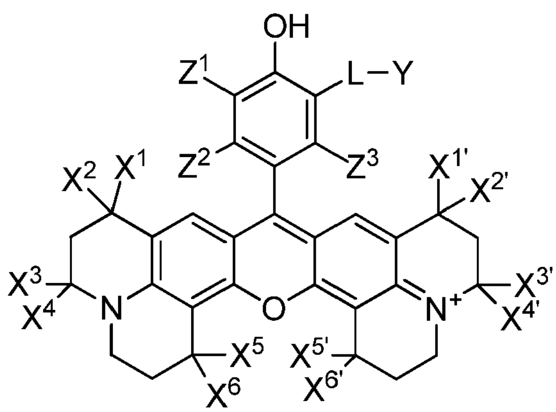

and salts thereof, wherein **L, Y**, **Z¹**, **Z**², **Z³**, **X¹**, **X**², **X³**, **X⁴**, **X⁵**, **X⁶**, **X¹'**, **X²'**, **X³'**, **X⁴', X⁵'** and **X⁶'** are as defined in claim **1.**

5. The compound according to anyone of claims **1** to **4,** of formula Ib:

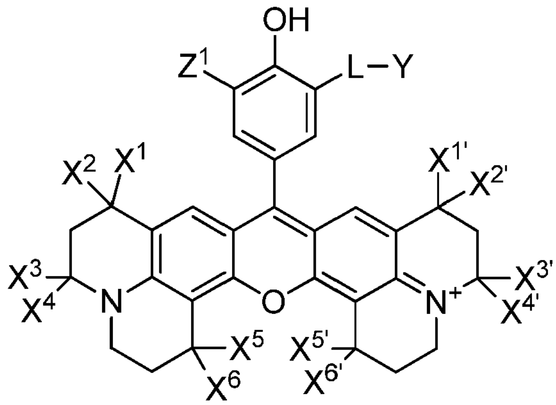

and salts thereof, wherein **L, Y, Z¹ X¹**, **X²**, **X³**, **X⁴, X⁵, X⁶**, **X¹'**, **X²'** X³', **X⁴'**, **X⁵'** and **X⁶'** are as defined in claim **1.**

6. The compound according to anyone of claims 1 to 5, of formula Ic:

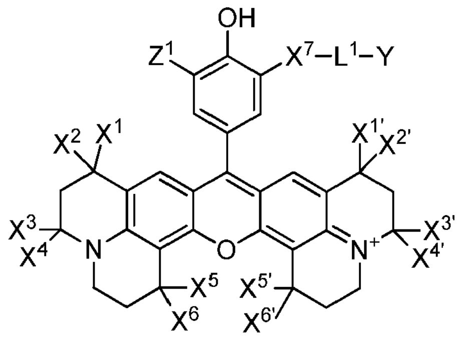

and salts thereof, wherein:

**Z¹, X¹**, **X²**, **X³**, **X⁴**, **X⁵, X⁶**, **X¹'**, **X²'**, **X³'**, **X⁴'**, **X⁵'**, **X⁶'** and **Y** are as defined in claim **1;**
**X⁷** represents a single bound or $-SO_2-$, -O-, -S-, -(C=O)N-, -(C=O)-; and
**L¹** represents a single bound, alkyl, aryl, alkylaryl, arylalkyl, or a combination thereof; optionally additionally comprising a residue of a reactive group through which L is bounded to **Y.**

7. The compound according to anyone of claims **1** to **6,** of formula Id:

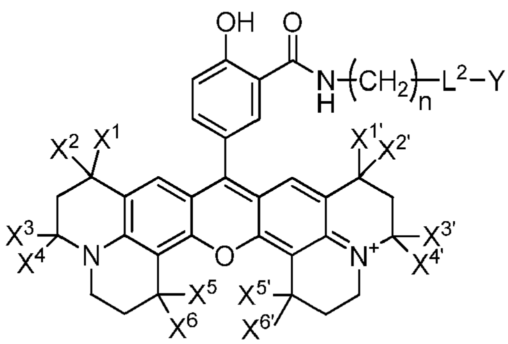

and salts thereof, wherein:

**$X^1$ $X^2$, $X^3$, $X^4$, $X^5$, $X^6$, $X^{1'}$**, **$X^{2'}$**, **$X^{3'}$**, **$X^{4'}$**, **$X^{5'}$**, **$X^{6'}$** and Y are as defined in claim **1;**
**$L^2$** represents a single bound or a residue of a reactive group through which Y is bounded to the rest of the compound; and
**n** represents an integer ranging from 1 to 8, preferably from 1 to 6, more preferably 1, 2, 3, or 4.

**8.** The compound according to anyone of claims 1 to 6, of formula Ie:

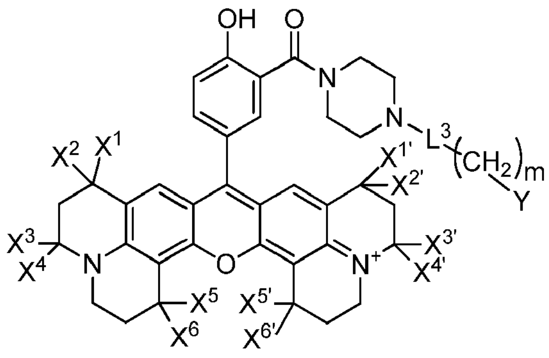

and salts thereof, wherein:

**$X^1$ $X^2$, $X^3$, $X^4$, $X^5$, $X^6$, $X^{1'}$**, **$X^{2'}$**, **$X^{3'}$**, **$X^{4'}$**, **$X^{5'}$**, **$X^{6'}$** and **Y** are as defined in claim **1;**
**$L^3$** represents a single bound or an alkyloxycabonyl; and
**m** represents an integer ranging from 1 to 8, preferably from 1 to 6, more preferably 1, 2, 3, or 4.

**9.** The compound according to anyone of claims **1** to **8,** selected from the group comprising compounds:

| | |
|---|---|
| **I-1** | 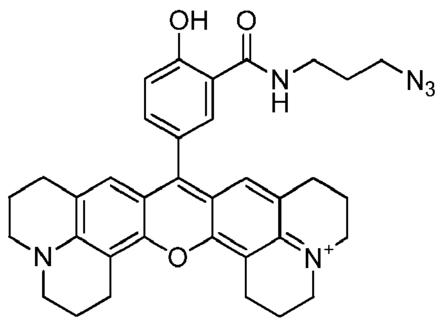 |
| **I-2** | 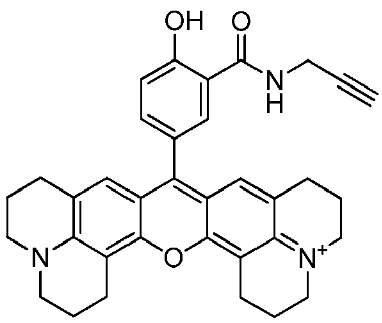 |

(continued)

| | |
|---|---|
| **I-3** | 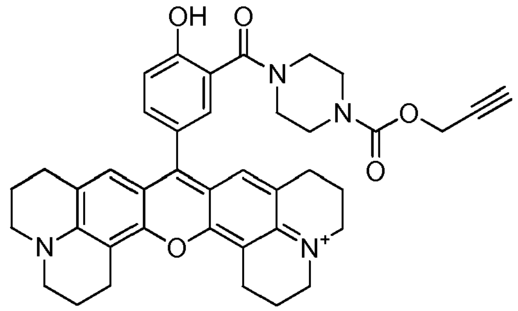 |
| **I-4** | 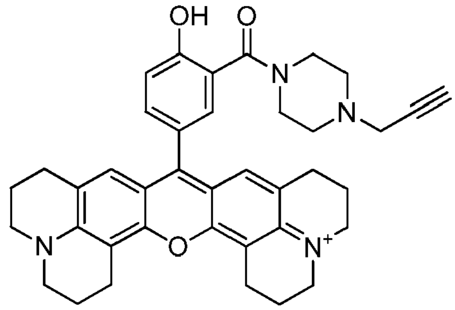 |
| **I-5** | 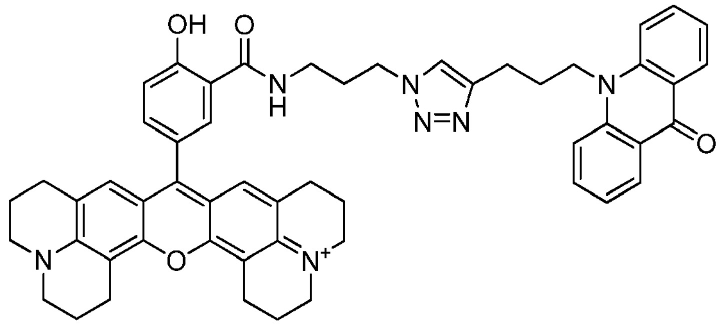 |
| **I-6** | 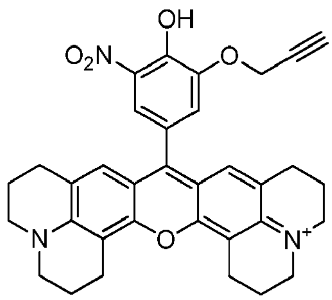 |

**10.** Process for manufacturing a compound of formula I:

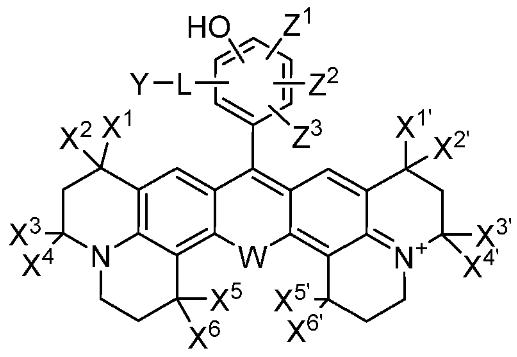

and salts thereof, wherein:

**W** represents O, NH, N(alkyl), Si(alkyl)$_2$, $CH_2$, CH(alkyl), C(alkyl)$_2$; preferably **W** represents O;
**X¹** and **X¹'** represent both H, both Me or both $-CH_2-SO_3H$;
**X²** and **X²'** represent both H or both Me;
**X³** and **X³'** represent both H or both Me;
**X⁴** and **X⁴'** represent both H or both Me;
**X⁵** and **X⁵'** represent both H or both Me;
**X⁶** and **X⁶'** represent both H or both Me;
**Z**$^1$, **Z**$^2$ and **Z**$^3$, represent each independently H, electron withdrawing group or electron donating group, preferably **Z**$^1$, **Z**$^2$ and **Z**$^3$, represent each independently H, alkyl, aryl, amino, alkylamino, dialkylamino, thiol, alkylthio, hydroxy, alkoxy, acylamino, (carboxyl ester)oxy, halo, $NO_2$, CN, $-SO_3H$, -COOH, ester, aldehyde, ketone;
**L** represents a single bound or a linker, preferably the linker is selected from aminocarbonyl, alkylaminocarbonyl, aminothiocarbonyl, alkyloxycarbonyl, alkyl, aryl, alkylaryl, arylalkyl, alkoxy, polyethylene glycol (PEG), polypropylene glycol (PPG), peptide or a combination thereof; optionally interrupted or terminated by -O-, -S-, $-SO_2$ or a combination thereof; optionally additionally comprising a residue of a reactive group through which L is bounded to **Y;**
**Y** represents:

- a reactive group selected from the group comprising $N_3$, amino, alkylamino, COOH, amide, maleimide, alkyne, SH, OH, ester, activated ester, activated carboxylic acid, halo, nitro, nitrile, isonitriles, acrylamide, aldehyde, ketone, acetals, ketals, anhydride, glutaric anhydride, succinic anhydride, maleic anhydride, thiocyanate, isothiocyanate, isocyanate, hydrazide, hydrazines, hydrazones, ethers, oxides, cyanates, diazo, diazonium, sulfides, disulfides, sulfoxides, sulfones, sulfonic acids, sulfinic acids, sulfates, sulfenic acids, amidines, imides, imidates, nitrones, hydroxylamines, oximes, hydroxamic acids, thiohydroxamic acids, allenes, ortho esters, sulfites, enamines, ynamines, ureas, pseudoureas, semicarbazides, carbodiimides, carbamates, imines;
- a bioactive group selected from amino acid, peptide, protein, antibody, enzyme, polysaccharide, dextran, benzylguanine, lipid, lipid assembly, fatty acid, nucleoside, nucleotide, oligonucleotide, hapten, aptamer, ligand, substrate, biotin, avidin, synthetic polymer, polymeric microparticle, nanoparticle, fluorophore, chromophore, radioisotope, macrocyclic complexes of radioisotope, and combinations thereof.

comprising a step of X-rhodamine formation on the aldehyde function of compound of formula II:

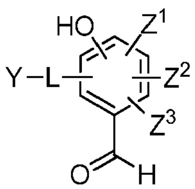

wherein **Z¹,** **Z**$^2$, **Z**$^3$, **L and Y** are as defined above;
to afford compound of formula I.

**11.** Use of a compound according to anyone of claims **1** to **9,** for the determination of pH.

**12.** Method for determining the pH of a sample comprising:

(a) contacting the sample with a compound according anyone of claims **1** to **9,** to form a contacted sample;
(b) incubating the contacted sample for an appropriate amount of time to form an incubated sample;
(c) illuminating the incubated sample at an exciting wavelength that generates a fluorescent response to form an illuminated sample; and
(d) detecting fluorescent emissions from the illuminated sample;

wherein the fluorescent emissions are used to determine the pH of the sample.

**13.** Method for monitoring the pH inside a live cell comprising:

(a) contacting the cell with a compound according anyone of claims **1** to **9** to form a contacted cell;
(b) incubating the contacted cell for an appropriate amount of time for the compound to enter the cell to form a labeled cell;
(c) illuminating the labeled cell at an exciting wavelength that generates a fluorescent response to form an illuminated cell; and
(d) detecting fluorescent emissions from the illuminated cell;

wherein the fluorescent emissions are used to monitor the pH inside the cell.

**14.** Kit for determining the pH of a sample comprising a compound according to anyone of claims **1** to **9.**

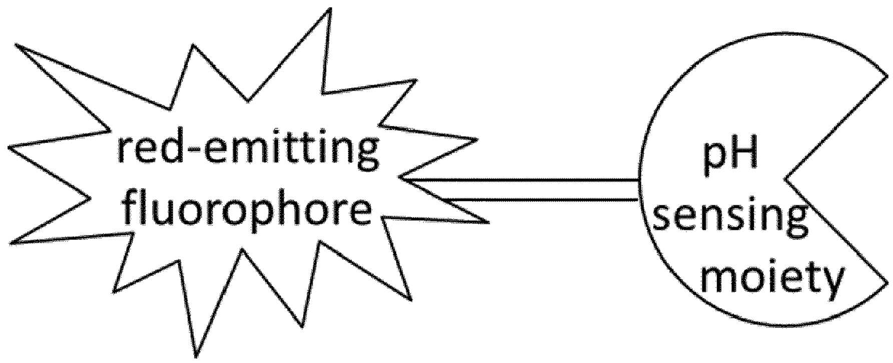


FIG. 1

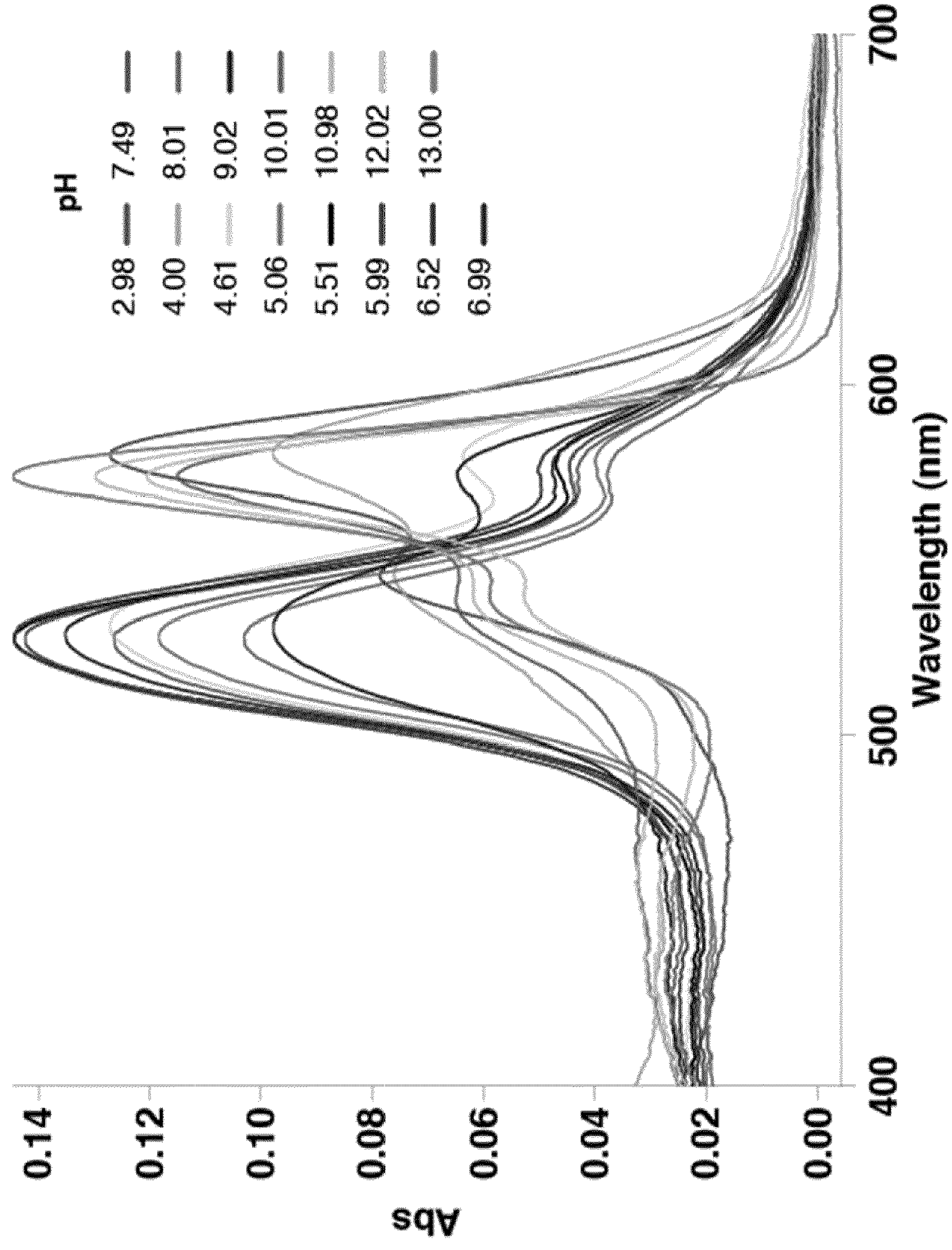
Abs
0.14
0.12
0.10
0.08
0.06
0.04
0.02
0.00
400
500
600
700
Wavelength (nm)
pH
2.98
4.00
4.61
5.06
5.51
5.99
6.52
6.99
7.49
8.01
9.02
10.01
10.98
12.02
13.00

FIG. 2

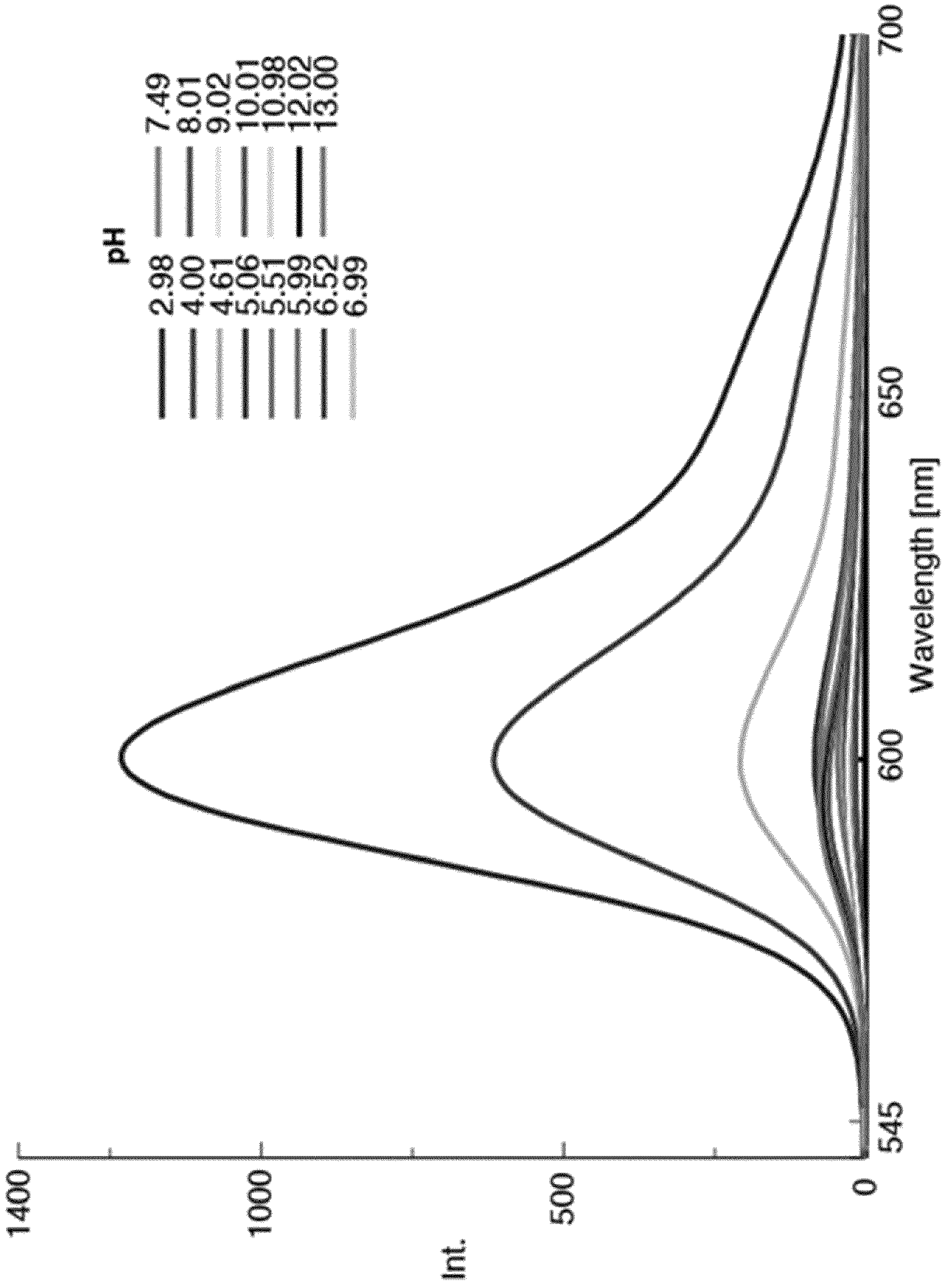
Int.
1400
1000
500
0
545
600
650
700
Wavelength [nm]
pH
2.98
4.00
4.61
5.06
5.51
5.99
6.52
6.99
7.49
8.01
9.02
10.01
10.98
12.02
13.00

**FIG. 3**

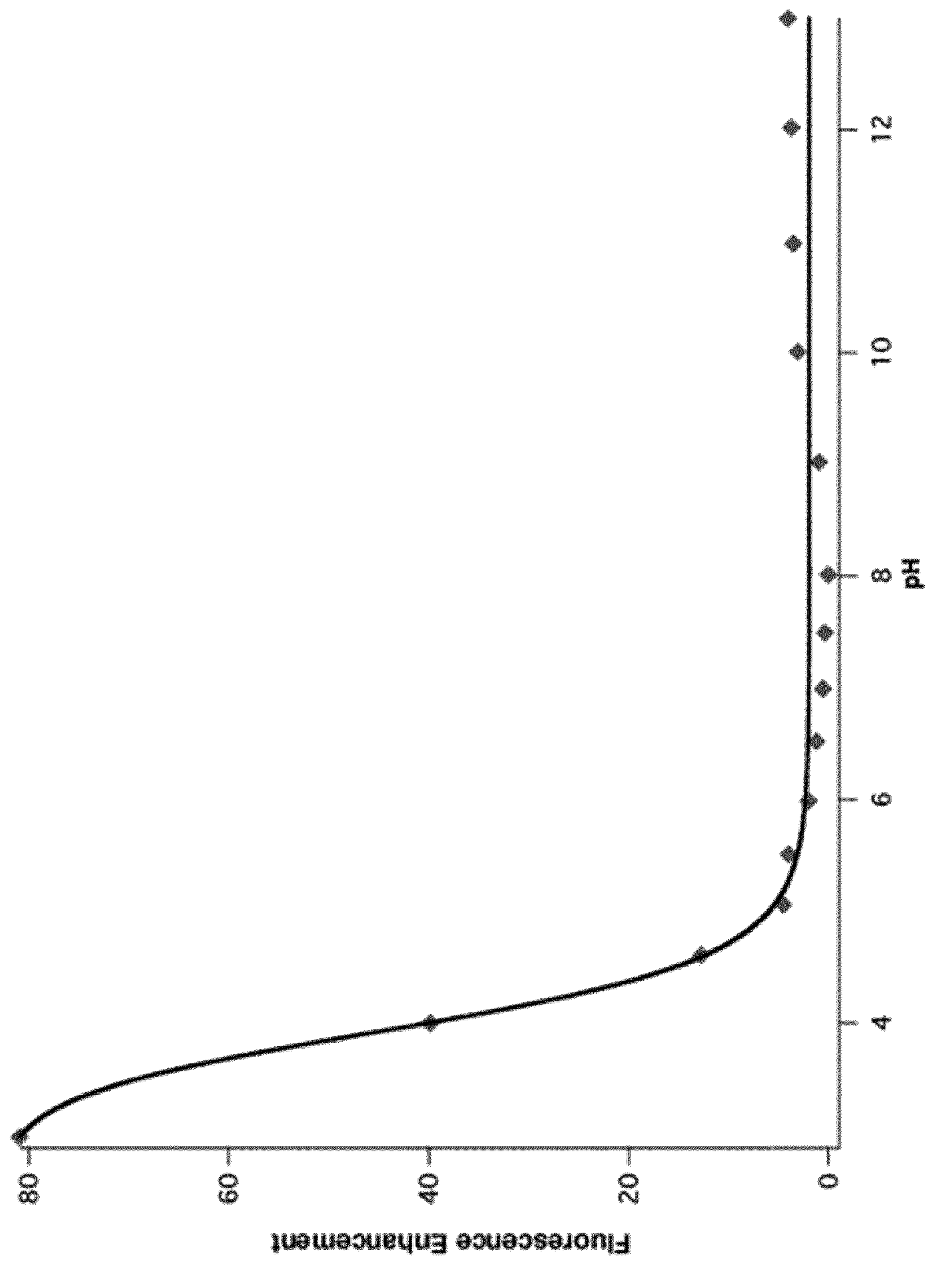


FIG. 4A

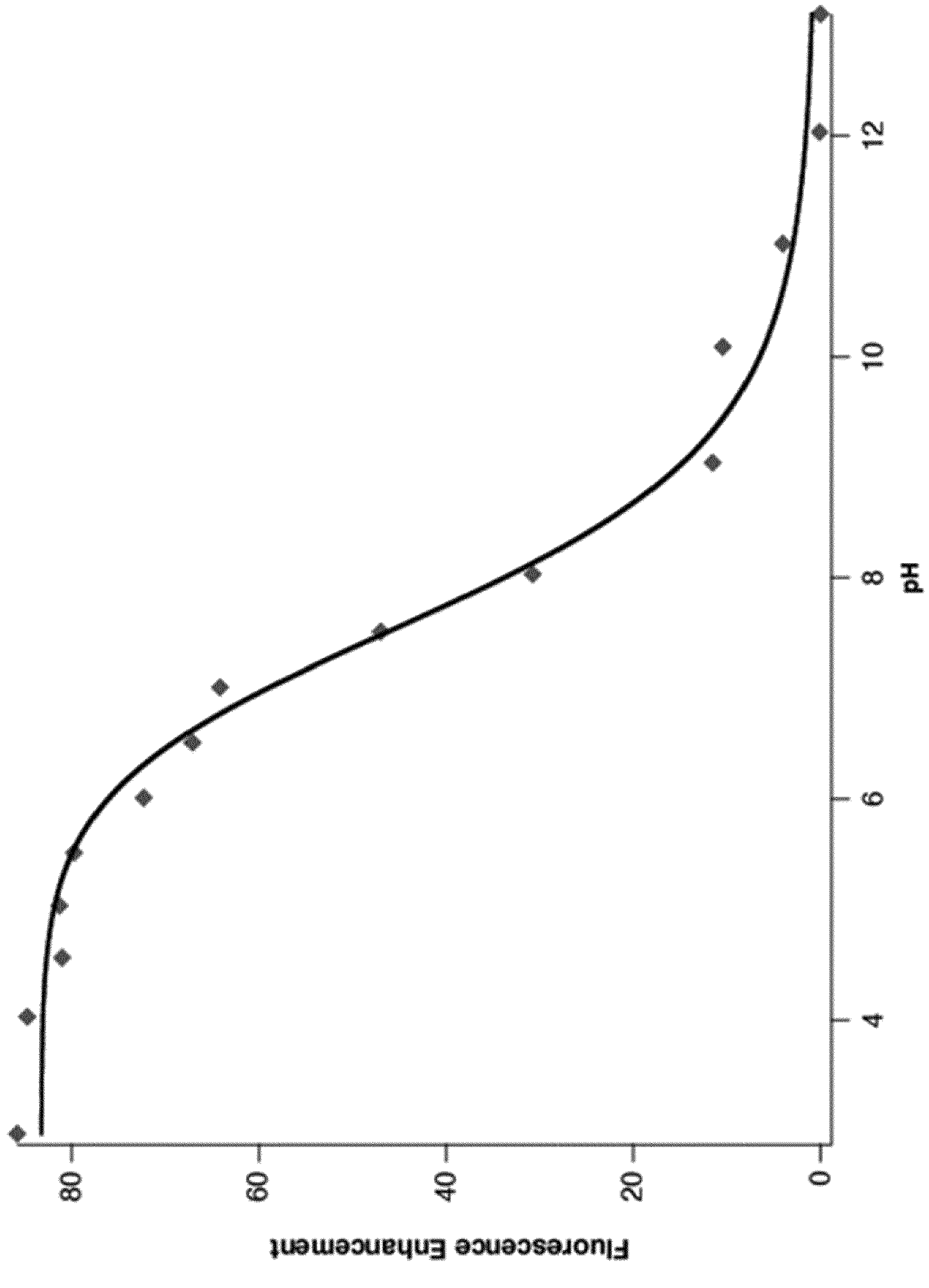


FIG. 4B

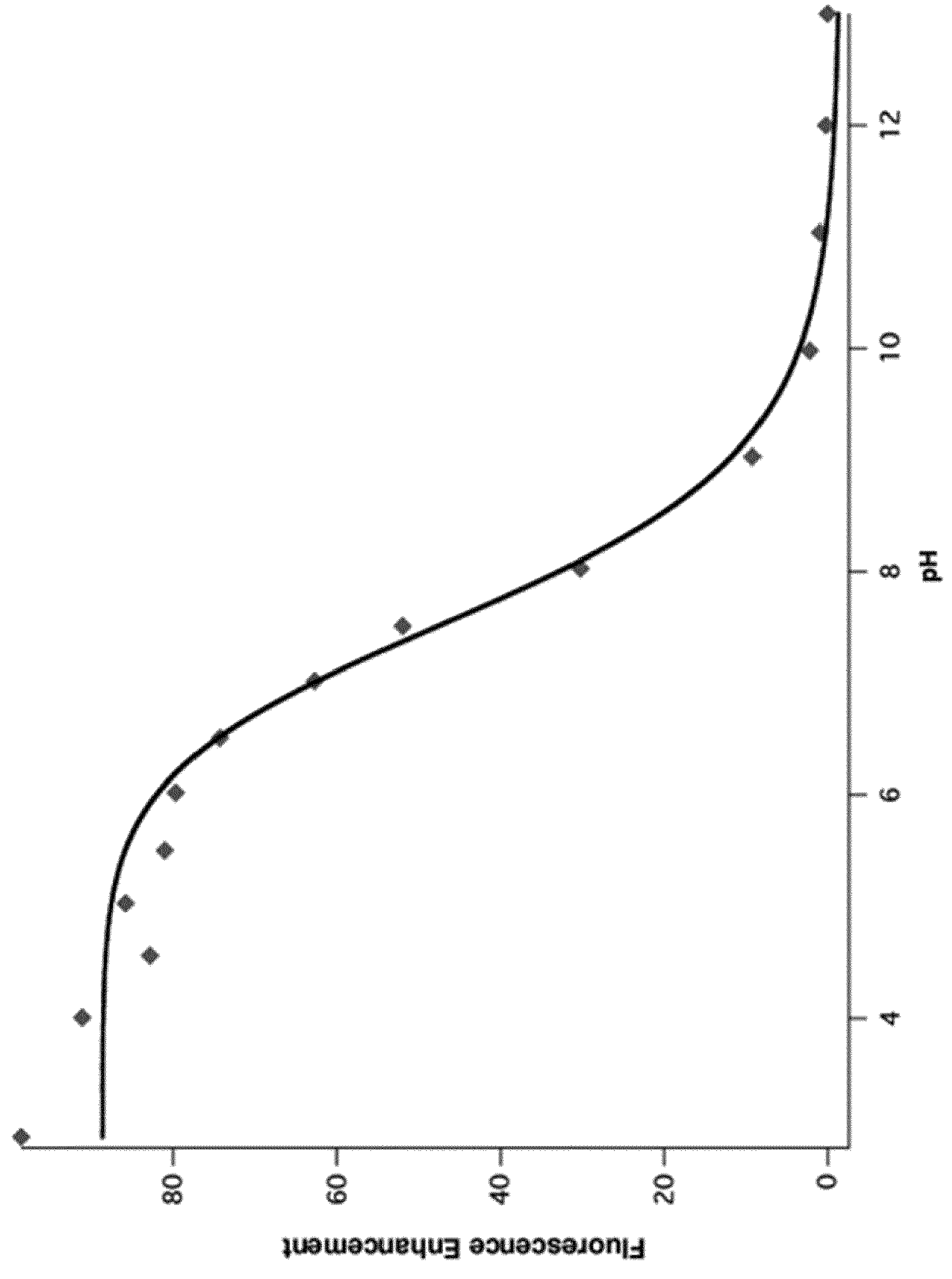


FIG. 4C

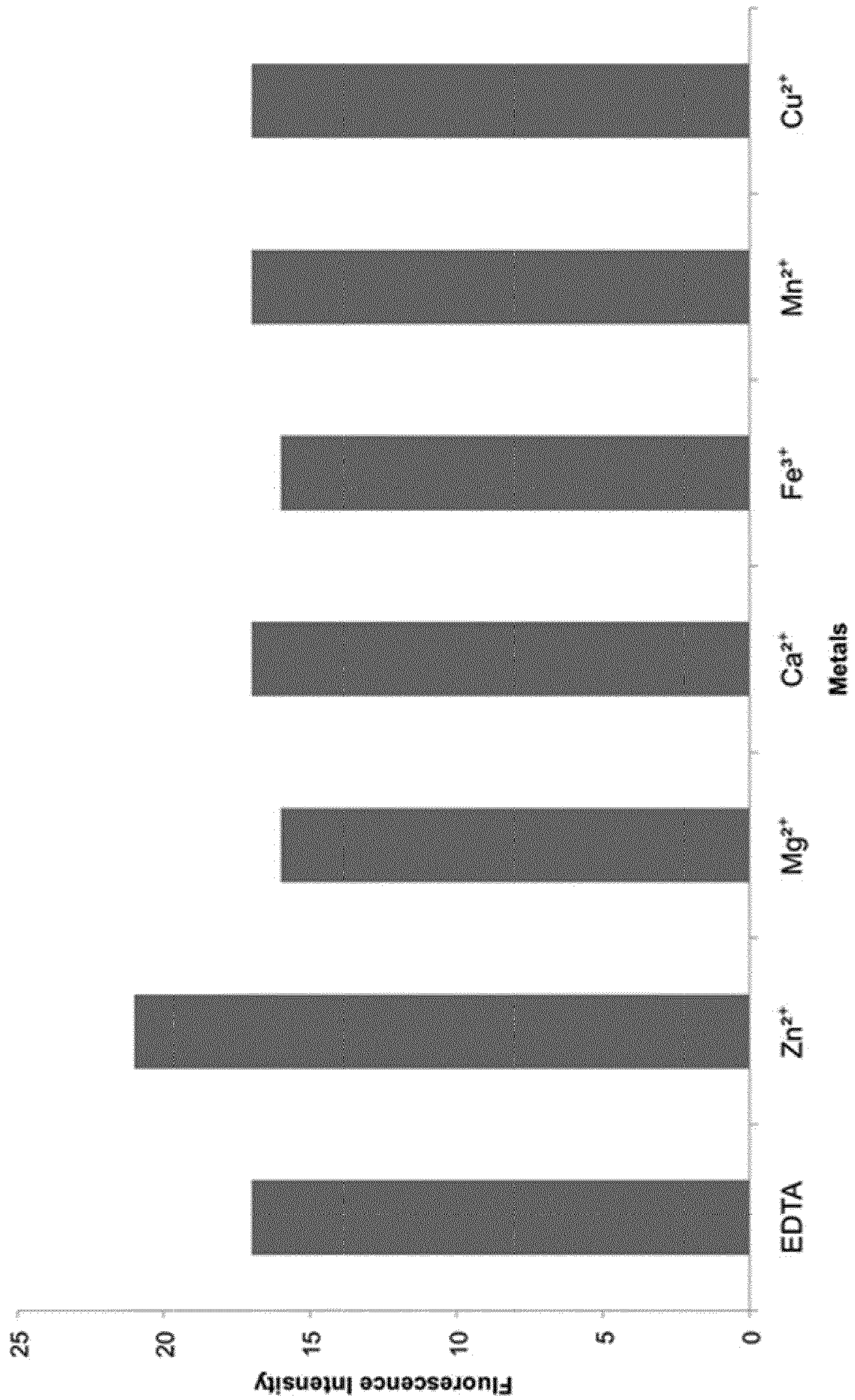
Fluorescence Intensity
25
20
15
10
5
0
EDTA
$Zn^{2+}$
$Mg^{2+}$
$Ca^{2+}$
$Fe^{3+}$
$Mn^{2+}$
$Cu^{2+}$
Metals

FIG. 5

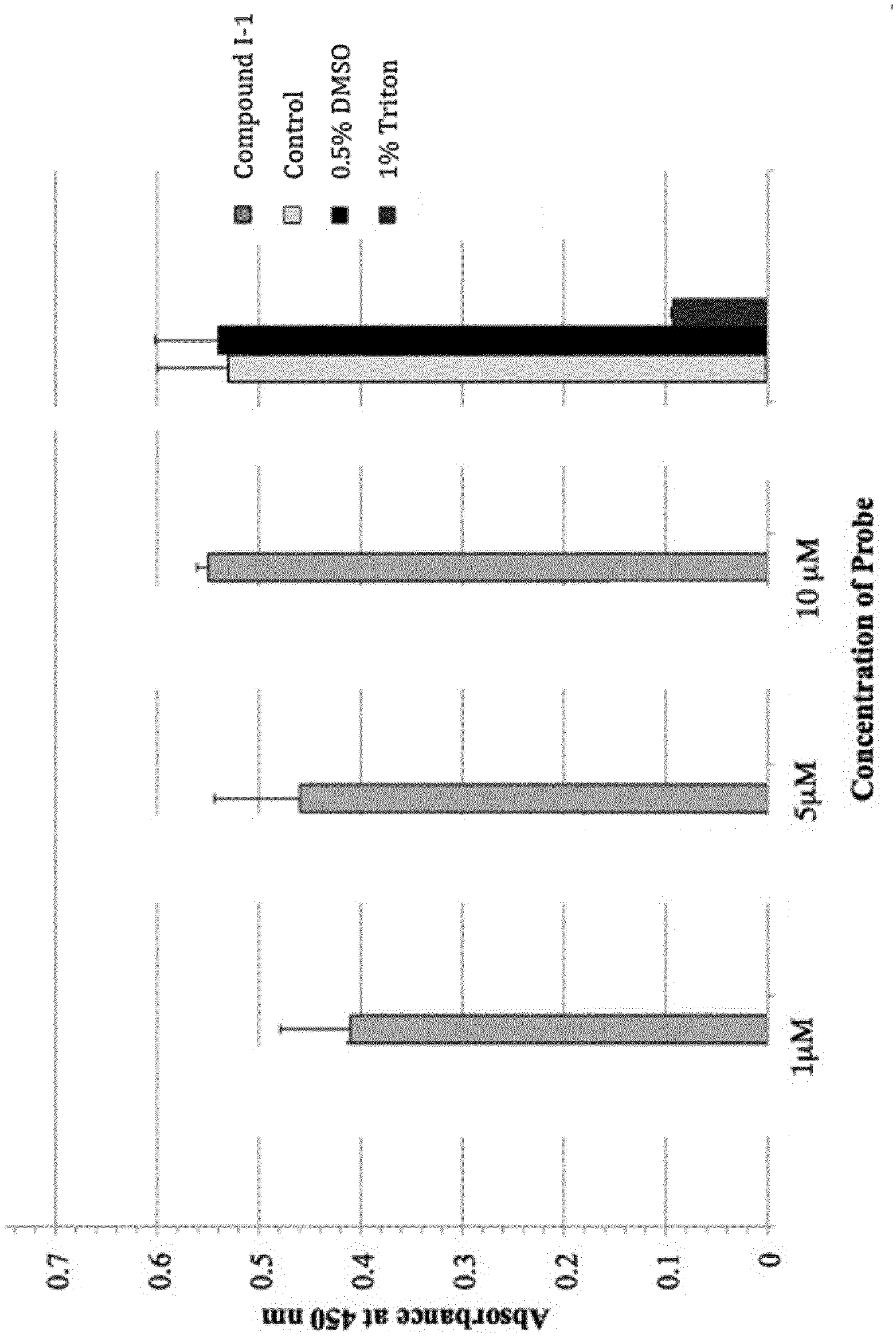
Compound I-1
Control
0.5% DMSO
1% Triton
Absorbance at 450 nm
0.7
0.6
0.5
0.4
0.3
0.2
0.1
0
1μM
5μM
10 μM
Concentration of Probe

FIG. 6

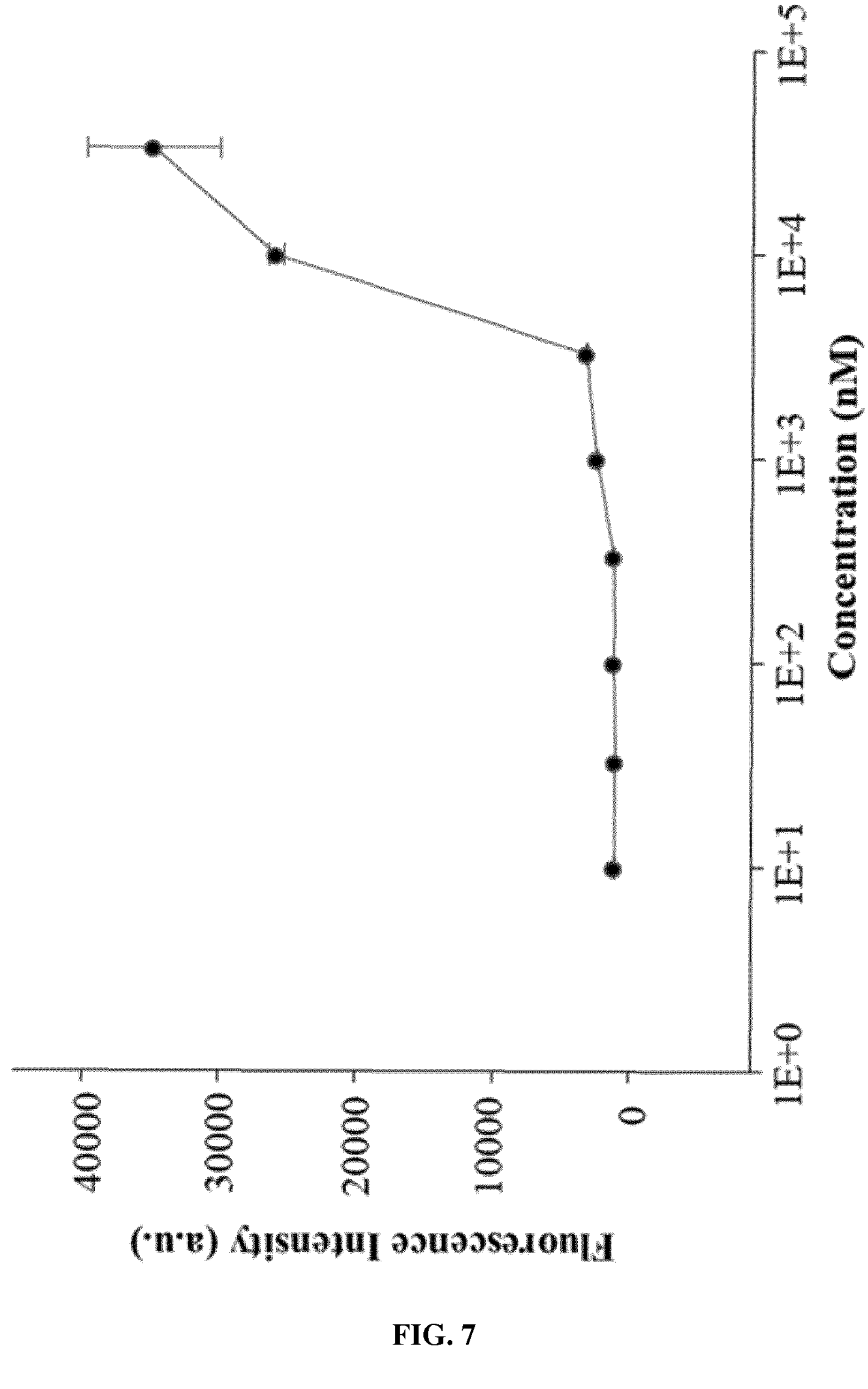
Fluorescence Intensity (a.u.)
40000
30000
20000
10000
0
1E+0
1E+1
1E+2
1E+3
1E+4
1E+5
Concentration (nM)

FIG. 7

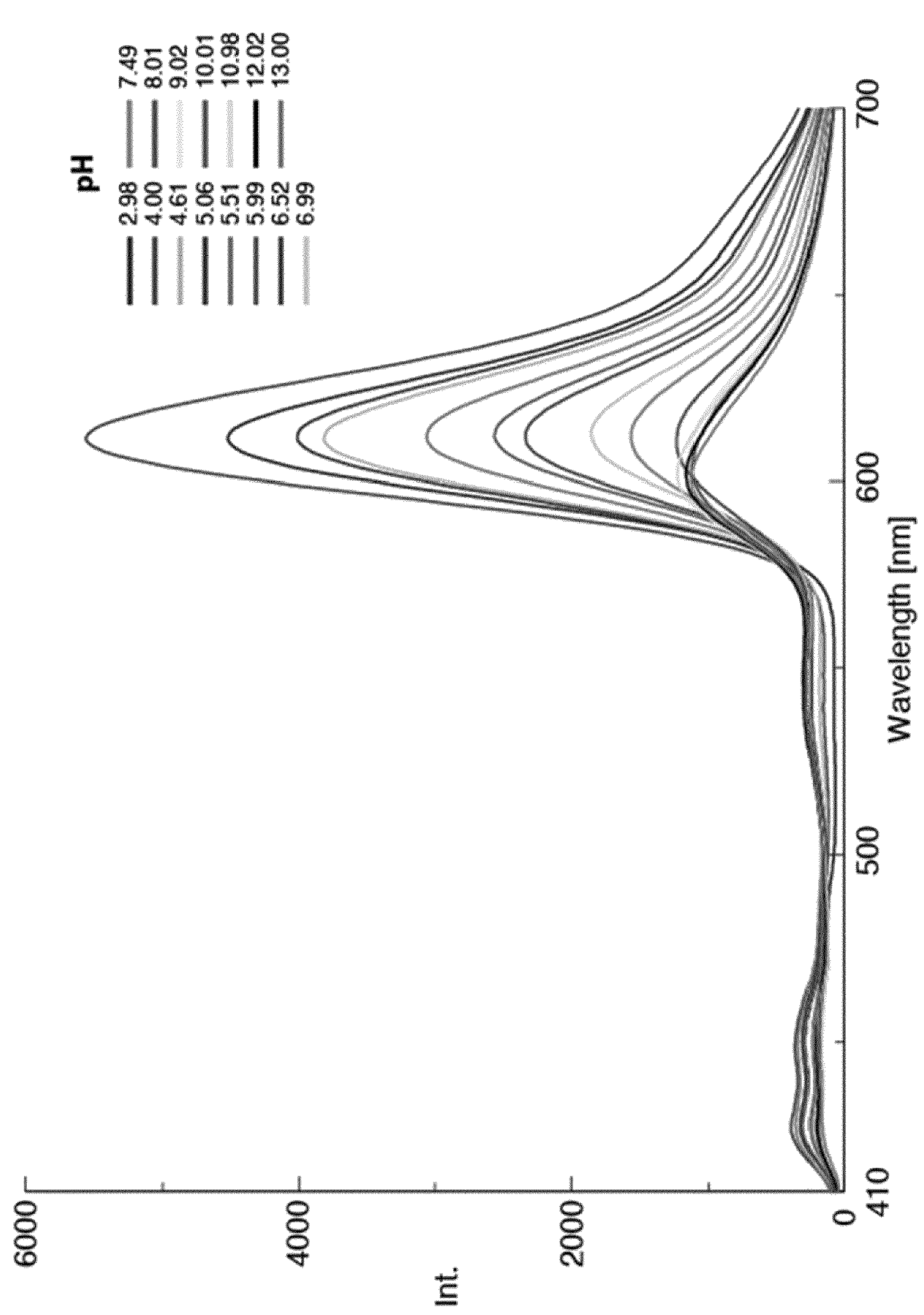
pH
2.98
4.00
4.61
5.06
5.51
5.99
6.52
6.99
7.49
8.01
9.02
10.01
10.98
12.02
13.00
Int.
6000
4000
2000
0
410
500
600
700
Wavelength [nm]

**FIG. 8**

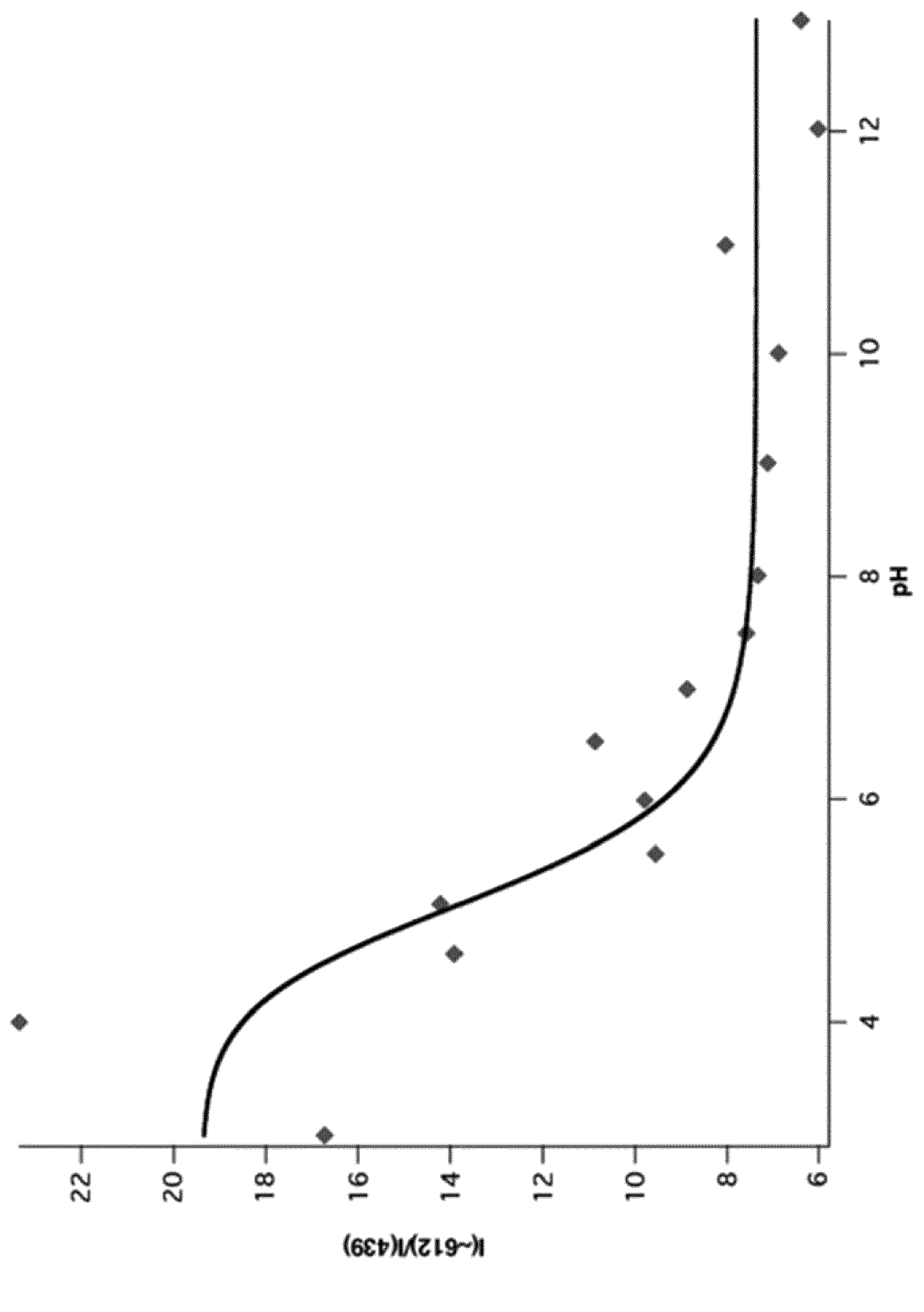


FIG. 9

Europäisches Patentamt
European Patent Office
Office européen des brevets

# EUROPEAN SEARCH REPORT

Application Number
EP 13 19 9575

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/076524 A2 (INVITROGEN CORP [US]; DZUBAY JEFFREY [US]; GEE KYLE [US]; MARTIN VLADI) 26 June 2008 (2008-06-26) | 1-3, 10-14 | INV. C07D491/00 C09B56/00 C09B62/00 |
| A | * compounds 155, 162 * | 4-9 | |
| | ----- | | |

TECHNICAL FIELDS SEARCHED (IPC)
C07D
C09B

The present search report has been drawn up for all claims

2

| Place of search | Date of completion of the search | Examiner |
|---|---|---|
| The Hague | 9 April 2014 | Hacking, Michiel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 19 9575

This annex lists the patent family members relating to the patent documents cited in the above-mentioned European search report. The members are as contained in the European Patent Office EDP file on
The European Patent Office is in no way liable for these particulars which are merely given for the purpose of information.

09-04-2014

| Patent document cited in search report | Publication date | Patent family member(s) | Publication date |
|---|---|---|---|
| WO 2008076524 A2 | 26-06-2008 | EP 2064290 A2 | 03-06-2009 |
| | | EP 2727965 A1 | 07-05-2014 |
| | | JP 2010508295 A | 18-03-2010 |
| | | US 2008274907 A1 | 06-11-2008 |
| | | US 2013102021 A1 | 25-04-2013 |
| | | WO 2008076524 A2 | 26-06-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008076524 A

### Non-patent literature cited in the description


- **HAN ; BURGESS.** *Chem. Rev.,* 2010, vol. 110, 2709-2728
- **TIAN et al.** *Dyes and Pigments,* 2012, vol. 95, 112-115
- **ZHANG et al.** *Analyst,* 2009, vol. 134, 367-371
- **BARUAH et al.** *J. Org. Chem.,* 2005, vol. 70, 4152-4157
- **QIN et al.** *ChemPhysChem,* 2005, vol. 6, 2343-2351
- **QIN et al.** *J. Photochem. Photobiol. A: Chem.,* 2006, vol. 183, 190-197
- **TAKEI et al.** *ChemComm,* 2013, vol. 49, 7313-7315